# EUROPEAN PATENT SPECIFICATION

(11) **EP 3 658 915 B1**
(45) Date of publication and mention of the grant of the patent: **05.03.2025**
(21) Application number: 18743530.0
(22) Date of filing: 26.07.2018
(51) Int. Cl.: G01N 33/574

(54) **NEW SUBPOPULATIONS OF CANCER ASSOCIATED FIBROBLASTS AS PROGNOSIS MARKERS FOR IMMUNOTHERAPY TREATMENTS**
NEUE SUBPOPULATIONEN VON TUMORASSOZIIERTEN FIBROBLASTEN ALS MARKER ZUR PROGNOSE VON IMMUNTHERAPIEBEHANDLUNGEN
NOUVELLES SOUS-POPULATIONS DE FIBROBLASTES ASSOCIÉS AU CANCER EN TANT QUE MARQUEURS DE PRONOSTIC POUR TRAITEMENTS IMMUNOTHÉRAPEUTIQUES

(30) Priority: 28.07.2017 EP 17306013
(43) Date of publication of application: 03.06.2020
(60) Divisional application: 25153407.9
(73) Proprietor: Institut Curie, 75005 Paris (FR); Institut National de la Santé et de la Recherche Médicale, 75013 Paris (FR)
(72) Inventor: COSTA, Ana, 75014 Paris (FR); KIEFFER, Yann, 94700 Maisons-Alfort (FR); GIVEL, Anne-Marie, 75018 Paris (FR); PELON, Floriane, 75012 Paris (FR); VINCENT-SALOMON, Anne, 75013 Paris (FR); MECHTA-GRIGORIOU, Fatima, 94100 Saint Maur Des Fosses (FR)
(74) Representative: Cabinet Becker et Associés
(86) International application number: PCT/EP2018/070248
(87) International publication number: WO 2019/020728

(56) References cited:
- WO-A1-2017/020974
- SAIGUSA SUSUMU ET AL: "Prognostic relevance of stromal CD26 expression in rectal cancer after chemoradiotherapy", INTERNATIONAL JOURNAL OF CLINICAL ONCOLOGY, CHURCHILL LIVINGSTONE JAPAN, TOKYO, GB, vol. 21, no. 2, 14 September 2015 (2015-09-14), pages 350 - 358, XP035894139, ISSN: 1341-9625, [retrieved on 20150914], DOI: 10.1007/S10147-015-0902-8
- ANGÉLICA M. SANTOS ET AL: "Targeting fibroblast activation protein inhibits tumor stromagenesis and growth in mice", JOURNAL OF CLINICAL INVESTIGATION, vol. 119, no. 12, 16 November 2009 (2009-11-16), US, pages 3613 - 3625, XP055423767, ISSN: 0021-9738, DOI: 10.1172/JCI38988
- ZHAOTING YANG ET AL: "Tenascin C is a prognostic determinant and potential cancer-associated fibroblasts marker for breast ductal carcinoma", EXPERIMENTAL AND MOLECULAR PATHOLOGY., vol. 102, no. 2, 20 February 2017 (2017-02-20), US, pages 262 - 267, XP055423771, ISSN: 0014-4800, DOI: 10.1016/j.yexmp.2017.02.012
- NI WEI-DONG ET AL: "Tenascin-C is a potential cancer-associated fibroblasts marker and predicts poor prognosis in prostate cancer", BIOCHEMICAL AND BIOPHYSICAL RESEARCH COMMUNICATIONS, ELSEVIER, AMSTERDAM, NL, vol. 486, no. 3, 21 March 2017 (2017-03-21), pages 607 - 612, XP029968953, ISSN: 0006-291X, DOI: 10.1016/J.BBRC.2017.03.021
- S BUSCH ET AL: "TGF-beta receptor type-2 expression in cancer-associated fibroblasts regulates breast cancer cell growth and survival and is a prognostic marker in pre-menopausal breast cancer", ONCOGENE, vol. 34, no. 1, 16 December 2013 (2013-12-16), pages 27 - 38, XP055423773, ISSN: 0950-9232, DOI: 10.1038/onc.2013.527
- YURI AKISHIMA-FUKASAWA ET AL: "Significance of PGP9.5 Expression in Cancer-Associated Fibroblasts for Prognosis of Colorectal Carcinoma", AMERICAN JOURNAL OF CLINICAL PATHOLOGY, vol. 134, no. 1, 1 July 2010 (2010-07-01), US, pages 71 - 79, XP055423774, ISSN: 0002-9173, DOI: 10.1309/AJCPRJP39MIDSGBH

## Description

### Field of the Invention

The present invention relates to the field of medicine, in particular of oncology. It provides new prognostic methods and markers for immunotherapy treatments.

### Background of the Invention

With 8.8 million deaths in 2015, cancer is the second leading cause of death worldwide, indeed nearly 1 in 6 deaths is due to cancer. The prevalence of cancer is also extremely high as more than 15 million new cases are diagnosed each year, and the number of new cases is expected to rise by about 70% over the next 2 decades. Among the most common cancers, lung cancers account for 1.69 million deaths per year, colorectal cancer for 774 000 deaths per year, and breast cancer for 571 000 deaths per year.

Many treatment options exist nowadays for cancer, including for example surgery, chemotherapy, radiation therapy, hormonal therapy, targeted therapy and palliative care. The choice of the best treatment depends on the type, location and grade of the cancer as well as the patient's health and preferences. However, there is still an important proportion of cancers, especially in late stage and/or metastatic cancers that remains resistant to treatment.

In the last few decades immunotherapy has become an important part of cancer treatment strategies. Cancer immunotherapy relies on the use of the immune system to treat cancer. Among the diversity of immunotherapy treatments that have been developed over time, immune checkpoint inhibitor therapies are particularly promising. However, not all cancers are responding with the same effectiveness to immunotherapy treatments. Indeed some cancers develop an immunosuppressive microenvironment that allow them to escape immunotherapy treatments.

There is thus still a strong need to identify new markers allowing to detect immunosuppressive environments in cancers and thus to predict the effectiveness of an immunotherapy treatment on a given cancer. That will allow to select immunotherapy treatments for patients who will really benefit from them. There is also a persistent need to develop new strategies to overcome immunosuppressive environments, thereby making immunotherapy treatments, and in particular immune checkpoint inhibitor treatments, more effective and available for all patients. The present invention seeks to meet these and other needs.

### Summary of the invention

Cancer is a systemic disease encompassing multiple components of both tumor cells themselves and host stromal cells. It is now clear that stromal cells in the tumor microenvironment play an important role in cancer development. Cancer stroma includes Cancer Associated Fibroblasts (CAFs), vascular endothelial cells, immune cells and the extracellular matrix. CAFs are the most frequent component of tumor stroma, they make up the bulk of cancer stroma and affect the tumor microenvironment such that they promote cancer initiation, angiogenesis, invasion and metastasis.

The present inventors have focused on Cancer Associated Fibroblasts (CAFs) and discovered new subpopulations of CAFs that play crucial roles in the establishment of an immunosuppressive microenvironment at the tumor site. Indeed, they have discovered that different subpopulations of CAFs act on adaptive T cell immunity by increasing the retention of CD4⁺CD25⁺ T-lymphocytes at the surface of stromal cells and by stimulating their activation state into CD25⁺FOXP3⁺ regulatory T-lymphocytes. CD25⁺FOXP3⁺ regulatory T-lymphocytes are known to be key actors in the establishment of immunosuppressive environments in tumors. Immunosuppressive environments are responsible of the development of resistances to cancer immunotherapy treatments, and in particular to immune checkpoints inhibitor treatments. The inventors identified markers that characterize CAF-mediated immunosuppression: DPP4, OX40L, CD73, PDL2 and B7H3. Indeed, they demonstrated that OX40L⁺ CAFs and PDL2⁺ CAFs, specifically increase the retention of CD4⁺CD25⁺ T-lymphocytes at the surface of stromal cells. They also showed that DPP4⁺ CAFs, CD73⁺ CAFs and B7H3⁺ CAFs specifically stimulate the activation of CD4⁺CD25⁺ T-lymphocytes into CD25⁺FOXP3⁺ regulatory T-lymphocytes.

The subject matter of the present invention is defined by the claims.

Accordingly, in a first aspect, the present invention concerns an *in vitro* method for selecting a patient affected with a tumor for an immune checkpoint inhibitor treatment, wherein the method comprises:
(a) detecting DPP4⁺ CAFs (Cancer Associated Fibroblast) in a cancer sample from said patient;
(b) determining the level of DPP4⁺ CAFs in said cancer sample, wherein the responsiveness of said patient to an immune checkpoint inhibitor treatment is inversely proportional to the level of DPP4⁺ CAFs in the cancer sample;
(c) selecting patients with level of DPP4⁺ CAFs lower than their reference level as suitable for an immune checkpoint inhibitor treatment, and/or selecting patients with a level of DPP4⁺ CAFs higher than their reference level for a cancer treatment excluding immune checkpoint inhibitor.

In a second aspect, the invention concerns an in vitro method for predicting the response of a subject affected with a solid tumor to an immune checkpoint inhibitor treatment, wherein the method comprises:
(a) detecting DPP4⁺ CAFs (Cancer Associated Fibroblast) in a cancer sample from said patient;
(b) determining the level of DPP4⁺ CAFs in said cancer sample, wherein the responsiveness of said patient to an immune checkpoint inhibitor treatment is inversely proportional to the level of DPP4⁺ CAFs in the cancer sample;
(c) comparing the level of DPP4⁺ CAFs in said cancer sample to a reference level, wherein level lower than their reference level is predictive of the responsiveness of said patient to an immune checkpoint inhibitor treatment, and/or level higher than their reference level is predictive of the inefficacy or lower efficacy of an immune checkpoint inhibitor treatment on the cancer of said patient.

Particularly, the method further comprises:
(a) detecting CD73+ CAFs, and/or B7H3+ CAFs, and/or OX40L+ CAFs, and/or JAM2+ CAFs in the cancer sample from said patient, and
(b) determining the level of CD73+ CAFs, and/or B7H3+ CAFs, and/or OX40L+ CAFs, and/or JAM2+ CAFs in said cancer sample, wherein the responsiveness of said patient to an immune checkpoint inhibitor treatment is inversely proportional to the levels of DPP4+ CAFs, and: CD73+ CAFs, and/or B7H3+ CAFs, and/or OX40L+ CAFs, and/or JAM2+ CAFs in the cancer sample,
(c) selecting patients with level of DPP4⁺ CAFs and: CD73+ CAFs, and/or B7H3+ CAFs, and/or OX40L+ CAFs, and/or JAM2+ CAFs lower than their reference level as suitable for an immune checkpoint inhibitor treatment, and/or selecting patients with a level of DPP4⁺ CAFs and: CD73+ CAFs, and/or B7H3+ CAFs, and/or OX40L+ CAFs, and/or JAM2+ CAFs higher than their reference level for a cancer treatment excluding immune checkpoint inhibitor.

In a further aspect, the invention also concerns an immune checkpoint inhibitor treatment for use in the treatment of a solid cancer in a patient wherein the patient has been selected for treatment by a method of the first aspect, wherein the patient presents in a cancer
(b) level(s) of CD73+ CAFs, and/or B7H3+ CAFs, and/or OX40L+ CAFs, and/or JAM2+ CAFs lower than their reference level.

Preferably, the immune checkpoint inhibitor treatment is selected from the group consisting of an anti-CTLA-4 (cytotoxic T lymphocyte associated protein 4) therapies such as ipilimumab, PD-1 (programmed cell death protein 1) inhibitors such as nivolumab, pembrolizumab, or BGB-A317, PDL1 (programmed cell death ligand) inhibitors such as atezolizumab, avelumab, or durvalumab, LAG-3 (Lymphocyte-activation gene 3) inhibitors such as BMS-986016, TIM-3 (T-cell immunoglobulin and mucin-domain containing-3) inhibitors, TIGIT (T cell immunoreceptor with Ig and ITIM domains) inhibitors, BLTA (B- and T-lymphocyte attenuator) inhibitors, IDO1 inhibitors such as epacadostat, or a combination thereof.

In particular, the anti-CTLA-4 therapy is ipilimumab, the PD-1 inhibitor is nivolumab, pembrolizumab, or BGB-A317, the PDL1 inhibitor is atezolizumab, avelumab, or durvalumab, the LAG-3 inhibitor is BMS-986016, the IDO1 inhibitor is epacadostat.

More preferably, the immune checkpoint inhibitor treatment is a PD-1 (programmed cell death protein 1) inhibitor or a PDL1 (programmed cell death ligand) inhibitor.

Preferably, in the invention, the level(s) of DPP4⁺ CAFs, in the cancer sample and the reference level(s) are reference percentage(s).

Preferably, the percentage of DPP4⁺ CAFs, in the cancer sample is calculated as the percentage of DPP4⁺ CAF cells, on the total number of cells in the cancer sample.

Preferably, the cancer is selected from the group consisting of prostate cancer, lung cancer, breast cancer, gastric cancer, kidney cancer, ovarian cancer, hepatocellular cancer, osteosarcoma, melanoma, hypopharynx cancer, esophageal cancer, endometrial cancer, cervical cancer, pancreatic cancer, liver cancer, colon or colorectal cancer, neuroendocrine tumors, muscle cancer, adrenal cancer, thyroid cancer, uterine cancer, skin cancer, bladder cancer, head and neck cancer, pediatric cancer, preferably the cancer is selected from the group consisting of ovarian cancer, breast cancer, lung cancer, colorectal cancer, pancreatic cancer and pediatric cancer.

More preferably, the cancer is an ovarian cancer, preferably a mesenchymal ovarian cancer, in particular high grade ovarian cancer of the serous type, or a breast cancer, preferably an invasive breast cancer and/or its metastasis, in particular axillary metastasis.

### Brief Description of the Drawings

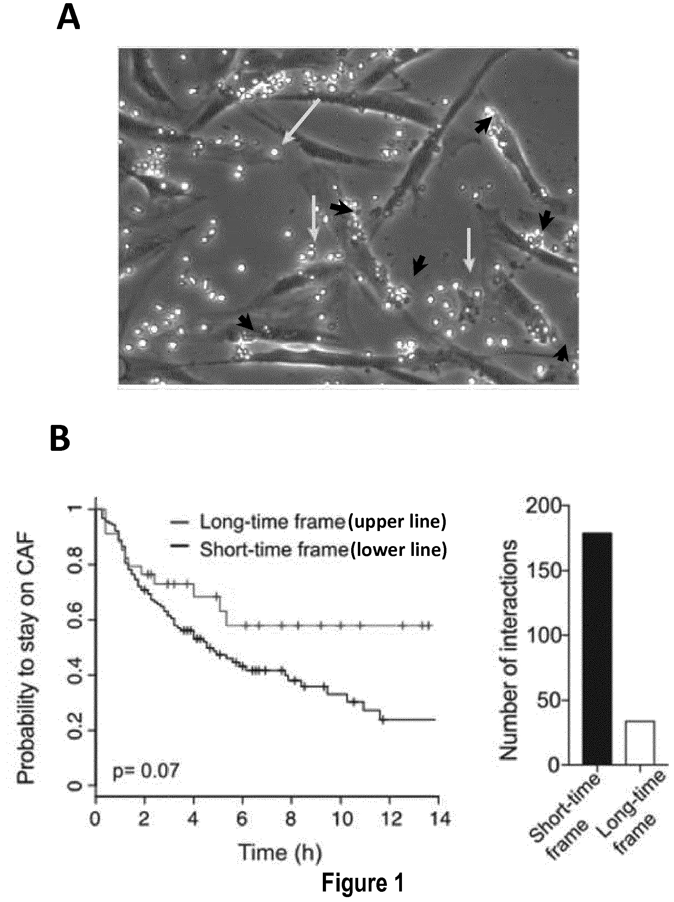

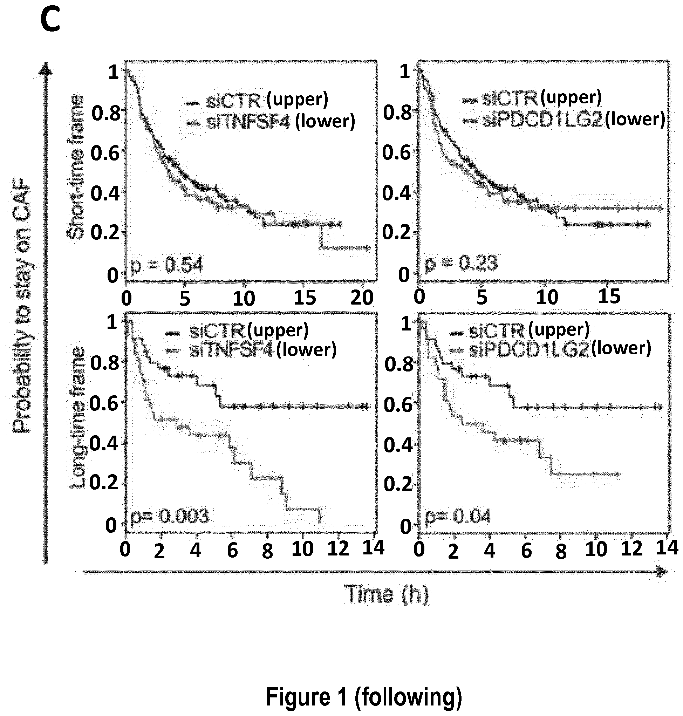

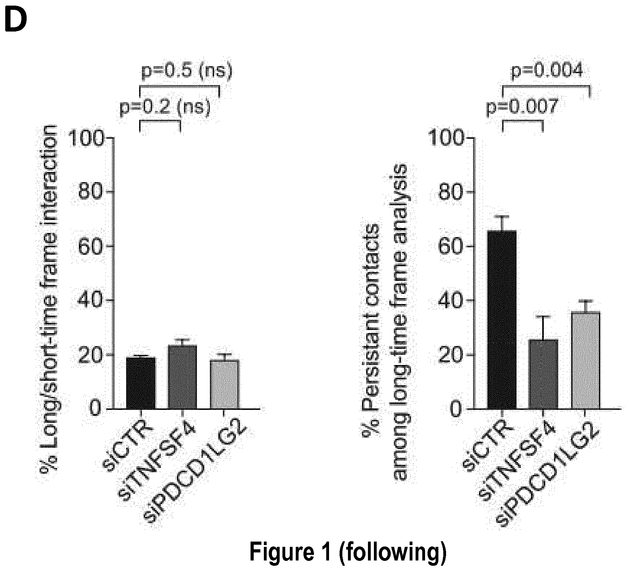

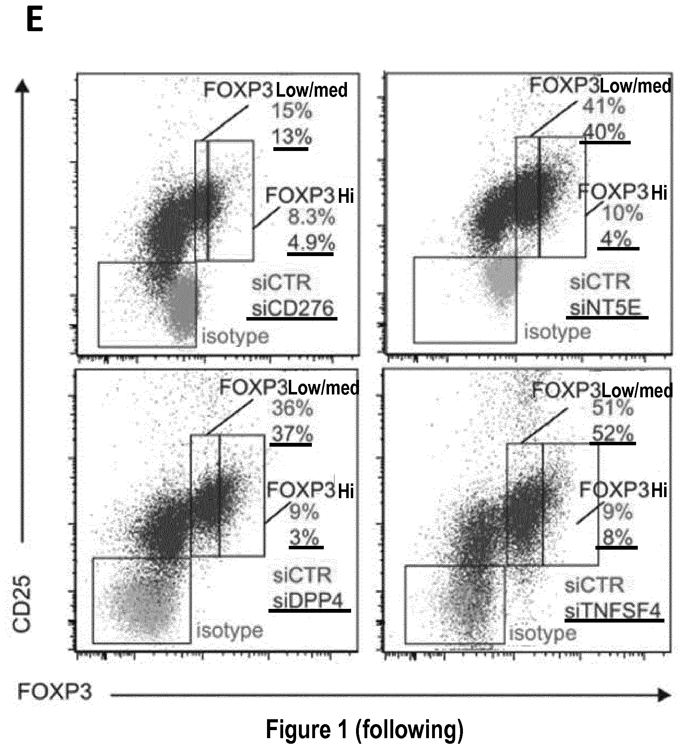

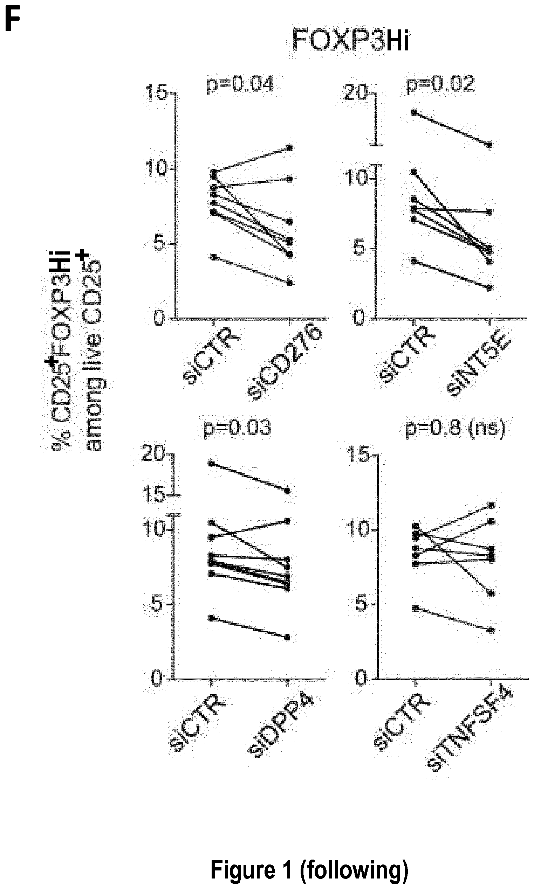

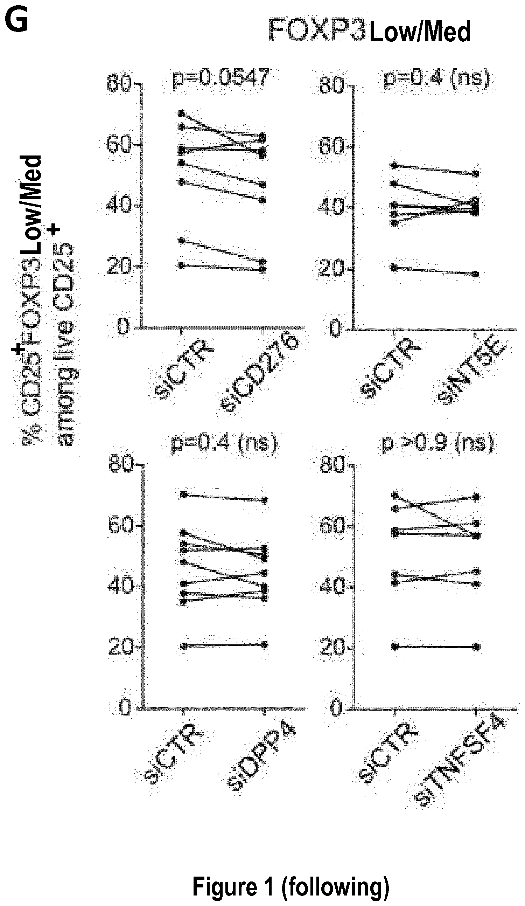

**Figure 1****: Mechanism of immunosuppression driven by CAFs**
   **A-D. Retention of CD4⁺CD25⁺ T-lymphocytes on CAFs through OX40L (TNFSF4) and PD-L2 (PDCD1LG2). A.** Representative photomicrograph of CAFs co-cultured with CD4⁺CD25⁺ T-lymphocytes showing two types of interaction of T cells with CAFs: short-time frame interactions (indicated by long arrows) and long-time frame interactions (indicated by short arrows). Magnification, 20X **B.** Kaplan-Meier curves of a representative experiment of CAFs transiently transfected with siCTR comparing short-time frame (lower line) and long-time frame (upper line) interactions between CAFs and CD4⁺CD25⁺ T-lymphocytes measured by the probability of T-lymphocytes to stay on CAFs (vertical axis) through time (h, hours, horizontal axis). P-value is from Log-rank test (Left). Number of interactions between CAFs and CD4⁺CD25⁺ T-lymphocytes considering short-time and long-time frames (Right). C. Kaplan-Meier curves from a representative experiment comparing CAFs transiently transfected with siCTR (upper line) and siTNFSF4 (OX40L) and siPDCD1LG2 (PD-L2) (lower lines) considering short-time frame (Top) or long-time frame (Bottom) interactions between CAFs and CD4⁺CD25⁺ T-lymphocytes, measured by the probability of T-lymphocytes to stay on CAFs (vertical axis) through time (h, hours, horizontal axis). P-value is from Log-rank test. D. Barplot showing frequency of stable interactions between CAFs transiently transfected with siCTR, siTNFSF4 (OX40L) and siPDCD1LG2 (PD-L2) and CD4⁺CD25⁺ T-lymphocytes (one representative experiment). Frequency of stable interactions is calculated as the ratio of the number of interactions considering long-time frame to the number of interactions considering short-time frame (Left). Barplot showing the percentage of persistent contacts among the long-time frame interactions between CAFs transiently transfected with siCTR, siTNFSF4 (OX40L) and siPDCD1LG2 (PD-L2) and CD4⁺CD25⁺ T-lymphocytes (Right). Error bars indicate mean ± s.e.m. *P*-values are based on Student t-test. **E-G. Activation of CD4⁺CD25⁺ T-lymphocytes into CD4+CD25+FOXP3+ regulatory T-lymphocytes by CAFs through CD276 (B7H3), NT5E (CD73) and DPP4. E.** Representative flow cytometry plots for CD25 and FOXP3 of CD4⁺CD25⁺ T-lymphocytes upon co-culture with CAFs transiently transfected with siCTR or siCD276 (B7H3), siNT5E (CD73) or siTNFSF4 (OX40L). Gating of CD25⁺FOXP3⁺ was determined according to the isotype (isotype box) and was divided into two classes according the level of FOXP3⁺ (FOXP3^{low/med} or POXP3^{high}). The percentages of T-lymphocytes in each gate are indicated for siCTR (not underlined) and for siCD276 (B7H3), siNT5E (CD73) or siTNFSF4 (OX40L) (underlined). **F.** Percentage of CD25⁺FOXP3⁺ T-lymphocytes among the CD25⁺ alive comparing CAFs transiently transfected with siCTR and siCD276, siNT5E, siDPP4 or siTNFSF4 for POXP3^{high}. **G.** Percentage of CD25⁺FOXP3⁺ T-lymphocytes among the CD25⁺ alive comparing CAFs transiently transfected with siCTR and siCD276, siNT5E, siDPP4 or siTNFSF4 for FOXP3^{low/med}. *P*-values were calculated by using the paired Wilcoxon signed-rank test. n ≥ 6 independent experiments.

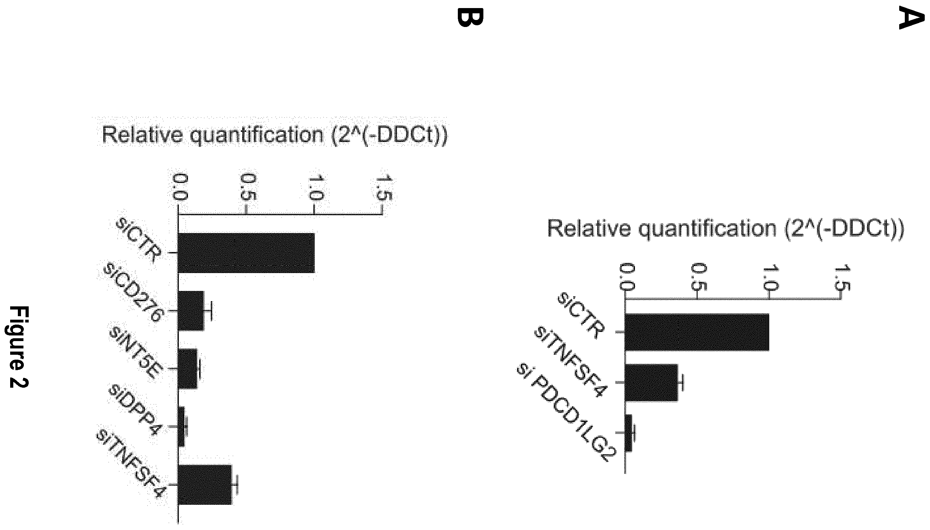

**Figure 2****: Efficiency of silencing by RT-qPCR**
   **A.** mRNA levels of TNFSF4 (OX40L) and PDCD1LG2 (PD-L2) in CAFs transiently transfected with siCTR, siTNFSF4 (OX40L) and siPDCD1LG2 (PD-L2) respectively, monitored by RT-qPCR. *Cyclophilin B* is used as internal control for total mRNA expression. Error bars indicate the s.e.m. (n≥4 independent experiments). **B.** mRNA levels of CD276 (B7H3), NT5E (CD73), DPP4 and TNFSF4 (OX40L) in CAFs transiently transfected with siCTR, siCD276 (B7H3), siNT5E (CD73), siDPP4 and siTNFSF4 (OX40L) respectively, monitored by RT-qPCR. *Cyclophilin B* is used as internal control for total mRNA expression. Error bars indicate the s.e.m. (n≥6 independent experiments).

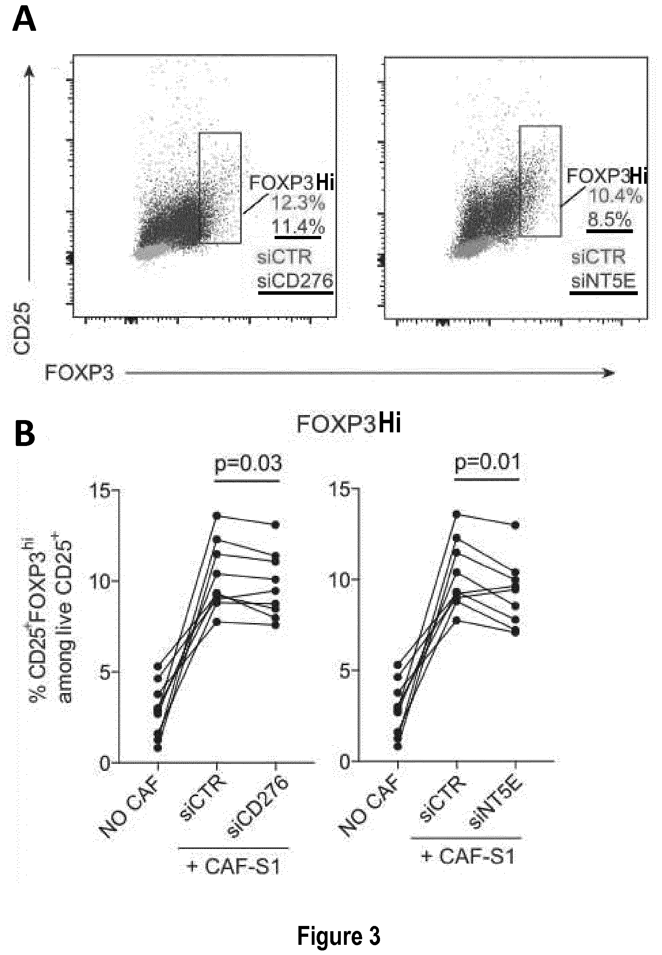

**Figure 3****: Mechanism of immunosuppression driven by CAFs in HGSOC**
   **Activation of CD4⁺CD25⁺ T-lymphocytes into CD4+CD25+FOXP3+ regulatory T-lymphocytes by CAFs through CD276 (B7H3) and NT5E (CD73) A.** Representative flow cytometry plots for CD25 and FOXP3 of CD4⁺CD25⁺ T-lymphocytes upon co-culture with CAFs transiently transfected with siCTR, siCD276 (B7H3) or siNT5E (CD73). Gating of CD25⁺FOXP3⁺ was determined according to the isotype and was divided into two classes according the level of FOXP3⁺ as in Figure 1E (FOXP3^{low/med} or FOXP3^{high}). The percentages of T-lymphocytes in FOXP3^{high} gate are indicated for siCTR (not underlined) and for siCD276 (B7H3) and siNT5E (CD73) (underlined). **B.** Percentage of CD25⁺FOXP3⁺ T-lymphocytes among the CD25⁺ alive comparing CAFs transiently transfected with siCTR and siCD276 and siNT5E for FOXP3^{high}. P-values were calculated by using the paired Wilcoxon signed-rank test. n ≥ 2 independent experiments.

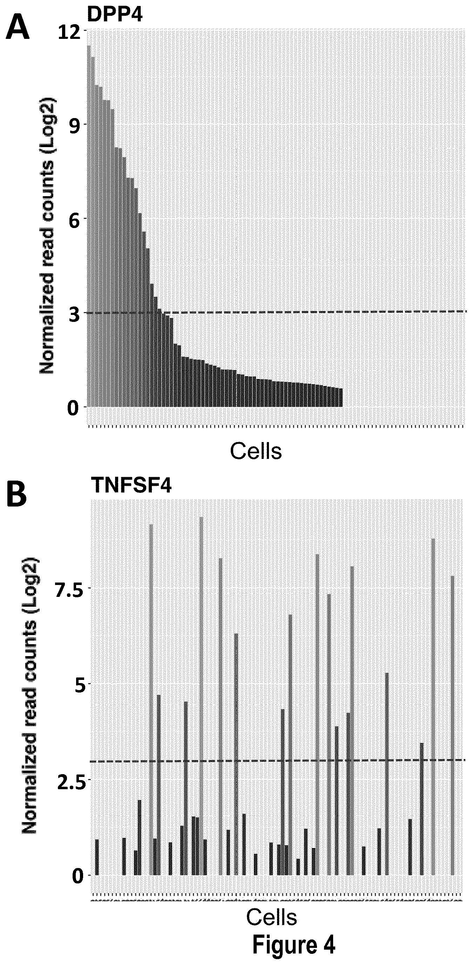

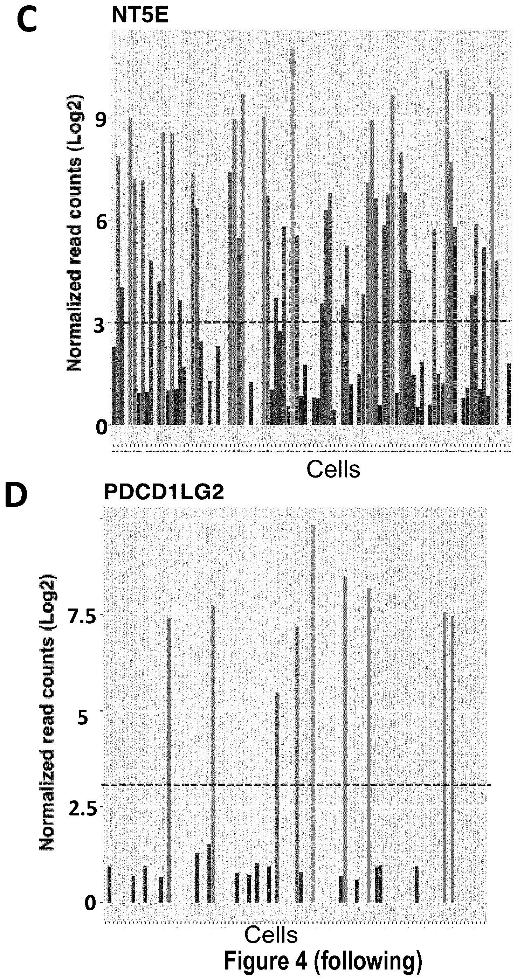

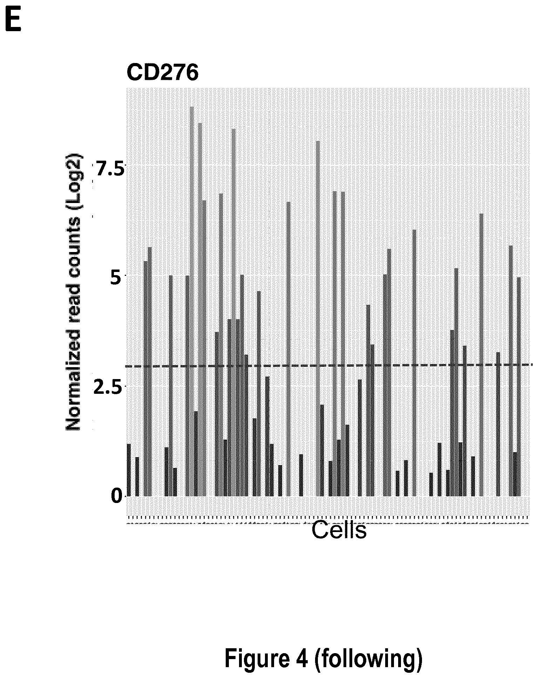

**Figure 4****: Identification of positive cells for each of the 5 markers**
   Barplots of normalized log-expression values for DPP4, OX40L (TNFSF4), CD73 (NT5E), PDL2 (PDCD1LG2) and B7H3 (CD276). Each bar represents the expression value for one cell. Cells with normalized expression higher than 3 (represented by the dashed line) were considered positive for the gene. Data are from RNA-Sequencing realized on 96 cells coming from 2 Breast Cancer (Lum A subtype) patients.

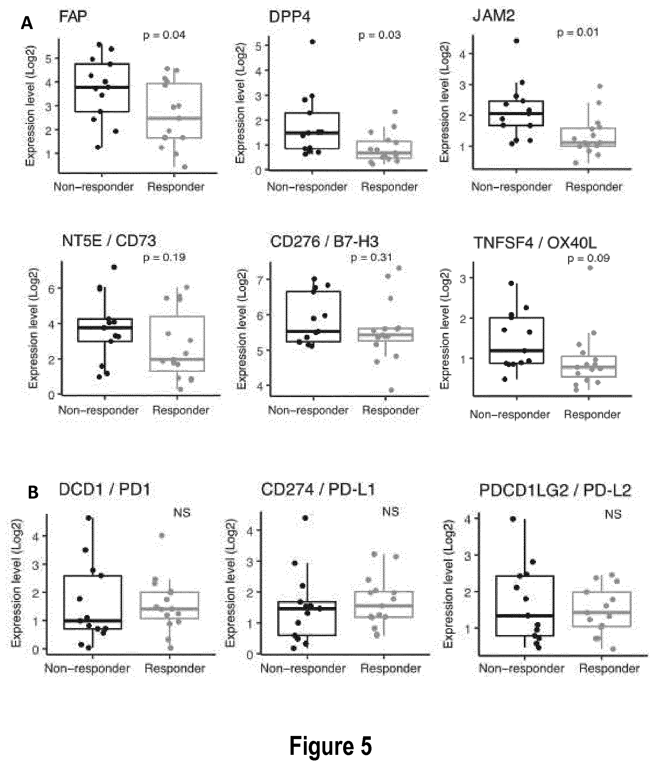

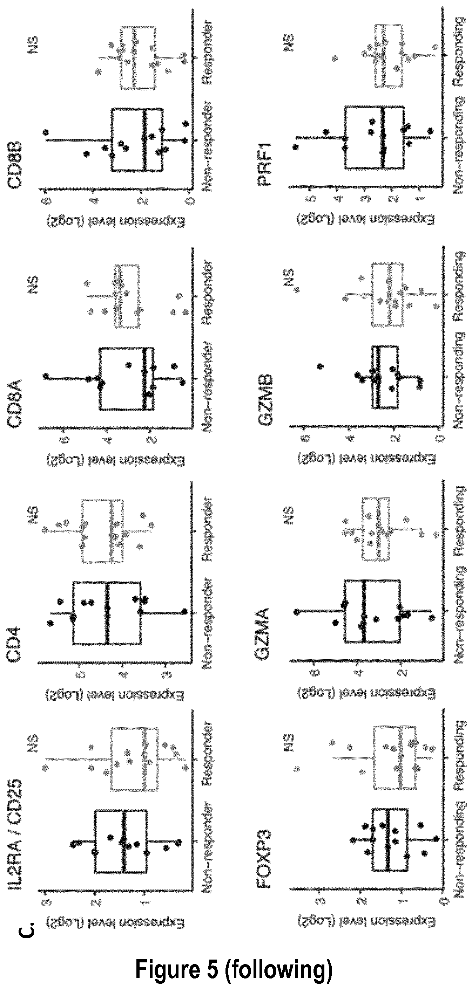

**Figure 5****: CAF-S1 markers involved in immunosuppression are indicative of response to immunotherapy while PD-1, PD-L1, PD-L2, CD25, CD4, CD8 T lymphocytes and markers of CD8 cytotoxicity are not.**
   A. Boxplots of normalized log-expression values for CAF-S1 markers involved in immunosuppression (FAP, DPP4, JAM2, OX40L, CD73 and B7-H3) according to response to anti-PD-1 therapy. B. Boxplots of normalized log-expression values for PD-1, PD-L1 and PD-L2 according to response to anti-PD-1 therapy. C. Boxplots of normalized log-expression values for CD25, CD4, CD8A, CD8B, FOXP3, GZMA, GZMB and PRF1 according to response to anti-PD-1 therapy. P-values are from Welch two-sample t-test.

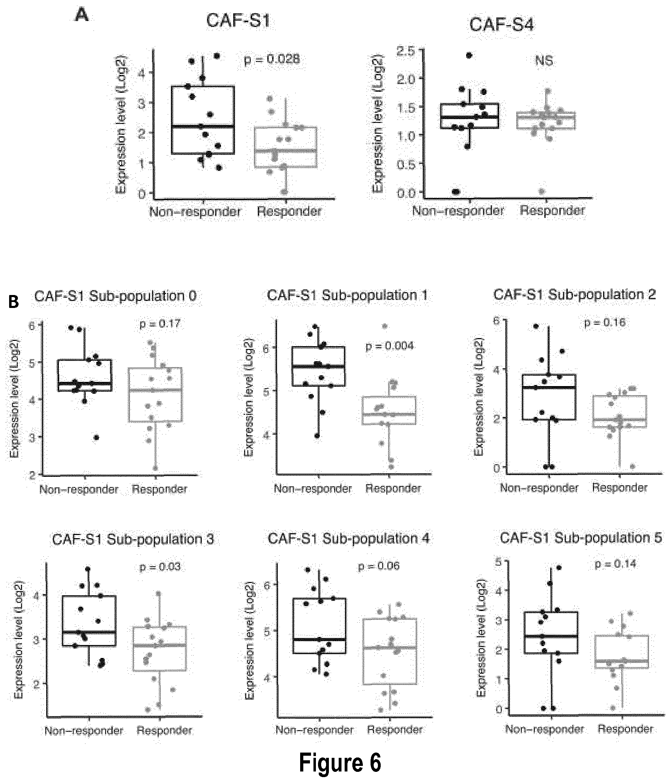

**Figure 6****: In contrast to CAF-S4, CAF-S1 signature is indicative of response to immunotherapy and especially two sub-populations of CAF-S1: sub-populations 1 and 3.**
   A. Boxplots of normalized log-expression scores for CAF-S1 gene signature (Left) and CAF-S4 gene signature (Right) according to response to anti-PD-1 therapy. B. Boxplots of normalized log-expression scores for 6 sub-populations of CAF-S1 according to response to anti-PD-1 therapy. P-values are from Welch two-sample t-test.

### Detailed description of the Invention

The inventors have discovered new subpopulations of Cancer Associated Fibroblasts (CAF) that play a crucial role in the establishment of an immunosuppressive microenvironment at the tumor site by increasing their retention of CD4⁺CD25⁺ T-lymphocytes at the surface of stromal cells and by stimulating their activation state into CD25⁺FOXP3⁺ regulatory T-lymphocytes. CD25⁺FOXP3⁺ regulatory T-lymphocytes are known to be key actors in the establishment of an immunosuppressive environment, which is responsible of the development of resistances to immunotherapy treatments. The inventors have thus identified five subpopulations of CAFs useful for characterizing an immunosuppressive environment: DPP4⁺ CAFs, CD73⁺ CAFs, and B7H3⁺ CAFs that specifically stimulate the activation of CD4⁺CD25⁺ T-lymphocytes into CD25⁺FOXP3⁺ regulatory T-lymphocytes, and OX40L⁺ CAFs and PDL2⁺ CAFs that specifically increase the retention of CD4⁺CD25⁺ T-lymphocytes at the surface of stromal cells.

### Definitions

The term "cancer" or "tumor", as used herein, refers to the presence of cells possessing characteristics typical of cancer-causing cells, such as uncontrolled proliferation, and/or immortality, and/or metastatic potential, and/or rapid growth and/or proliferation rate, and/or certain characteristic morphological features. This term refers to any type of malignancy (primary or metastases) in any type of subject. It may refer to solid tumor as well as hematopoietic tumor.

The term "cancer sample", as used herein, means any sample containing tumoral cells and cancer stromal cells, in particular Cancer Associated Fibroblasts (CAF), derived from a subject. Preferably the cancer sample contains nucleic acids and/or proteins. The sample may be treated prior to its use.

Cancer tissues are composed of cancer cells and the surrounding cancer stromal cells, including cancer associated fibroblasts (CAFs), vascular endothelial cells, and immune cells, in addition to the extracellular matrix. As used herein, the term "Cancer Associated Fibroblast" or "CAF" refers to the fibroblasts present in the stroma of cancers. CAFs are CD45⁻EpCAM⁻CD31⁻CD29⁺ stromal cells. CAFs are one of the most abundant stromal components with a morphology similar to that of myofibroblasts.

As used herein, the terms "subject", "individual" or "patient" are interchangeable and refer to an animal, preferably to a mammal, even more preferably to a human. However, the term "subject" can also refer to non-human animals, in particular mammals such as dogs, cats, horses, cows, pigs, sheep and non-human primates, among others.

As used herein, the term "marker" or "biomarker" refers to a measurable biological parameter that helps to predict the occurrence of a cancer or the efficiency of a cancer treatment.

As used herein, the term "diagnosis" refers to the determination as to whether a subject is likely to be affected by a cancer. The skilled artisan often makes a diagnosis on the basis of one or more diagnosis markers, the presence, absence, or amount of which is indicative of the presence or absence of the cancer. By "diagnosis", it is also intended to refer to the provision of information useful for diagnosis.

As used herein, the term "poor prognosis" refers to a decreased patient survival and/or an early disease progression and/or an increased disease recurrence and/or an increased metastasis occurrence.

As used herein, the term "treatment", "treat" or "treating" refers to any act intended to ameliorate the health status of patients such as therapy, prevention, prophylaxis and retardation of the disease. In certain embodiments, such term refers to the amelioration or eradication of a disease or symptoms associated with a disease. In other embodiments, this term refers to minimizing the spread or worsening of the disease resulting from the administration of one or more therapeutic agents to a subject with such a disease.

As used herein, the term "immunotherapy" refers to a cancer therapeutic treatment using the immune system to reject cancer. The therapeutic treatment stimulates the patient's immune system to attack the malignant tumor cells. It includes immunization of the patient with tumoral antigens (e.g. by administering a cancer vaccine), in which case the patient's own immune system is trained to recognize tumor cells as targets to be destroyed, or administration of molecules stimulating the immune system such as cytokines, or administration of therapeutic antibodies as drugs, in which case the patient's immune system is recruited by the therapeutic antibodies to destroy tumor cells. In particular, antibodies are directed against specific antigens such as the unusual antigens that are presented on the surfaces of tumors.

An important part of the immune system is its ability to tell between normal cells in the body and those it sees as "foreign", in particular cancer cells. This lets the immune system attack the cancer cells while leaving the normal cells alone. To do this, the immune system uses "checkpoints", these checkpoints are molecules on certain immune cells that need to be activated (or inactivated) to start an immune response. Cancer cells sometimes find ways to use these checkpoints to avoid being attacked by the immune system. As used herein, the term "immune checkpoint inhibitor treatment" refers to an immunotherapy treatment that target these checkpoints in order to allow or facilitate the attack of cancer cells by the immune system.

The terms "quantity," "amount," and "level" are used interchangeably herein and may refer to an absolute quantification of a molecule in a sample, or to a relative quantification of a molecule in a sample, i.e., relative to another value such as relative to a reference value as taught herein.

As used herein, the terms "active principle", "active ingredient" "active pharmaceutical ingredient", "therapeutic agent", "antitumor compound", and "antitumor agent" are equivalent and refer to a component having a therapeutic effect.

As used herein, the term "therapeutic effect" refers to an effect induced by an active ingredient or by a pharmaceutical composition according to the disclosure, capable to prevent or to delay the appearance or the development of a cancer, or to cure or to attenuate the effects of a cancer.

As used herein, the term "effective amount" refers to a quantity of an active ingredient which prevents, removes or reduces the deleterious effects of the disease.

In the present document, the term « about » refers to a range of values of ± 10% of the specified value. For example, « about 50 » comprise values of ± 10% of 50, i.e. values in the range between 45 and 55. Preferably, the term « about » refers to a range of values of ± 5% of the specified value.

As used herein, the terms "Dipeptidyl Peptidase 4", "DPP4", "DPPIV", "CD26", "ADABP", "ADCP2", or "TP103" are equivalent and refer to the product of the human DPP4 gene (Gene ID: 1803, UniGene Hs. 368912). This protein is a cell surface glycoprotein receptor involved in the costimulatory signal essential for T-cell receptor (TCR)-mediated T-cell activation (UniProt accession number: P27487). The GenBank entry of the sequence of the mRNA of the human DPP4 protein is M80536.1.

As used herein, the terms "OX40 Ligand", "OX40L", "Tumor necrosis factor ligand superfamily member 4", "TNFSF4", "TAX transcriptionally-activated glycoprotein 1", "TXGP1", "GP34", "CD134L", "TNLG2B", or "CD252" are equivalent and refer to the product of the human OX40L gene (Gene ID: 7292, UniGene Hs.181097). This protein is a cytokine that binds to TNFRSF4 (UniProt accession number: P23510). The GenBank entry of the sequence of the mRNA of the human OX40L protein is D90224.1.

As used herein, the terms "5'-nucleotidase (EC: 3.1.3.5)", "Ecto 5'-nucleotidase", "5'-NT", "NT5E", "CD73", "NT5", or "NTE" are equivalent and refer to the product of the human NT5E gene (Gene ID: 4907, UniGene Hs.153952). This protein hydrolyzes extracellular nucleotides into membrane permeable nucleosides (UniProt accession number: P21589). The GenBank entry of the sequence of the mRNA of the human NT5E protein is X55740.1.

As used herein, the terms "Programmed cell death 1 ligand 2", "PDCD1LG2", "Programmed cell death 1 ligand 2", "PDL2", "PD-1 ligand 2", "Butyrophilin B7-DC", or "CD273" are equivalent and refer to the product of the human PDCD1LG2 gene (Gene ID: 80380, UniGene Hs.532279). This protein is involved in the costimulatory signal of T-cell (UniProt accession number: Q9BQ51). The GenBank entry of the sequence of the mRNA of the human PDCD1LG2 protein is AF329193.1.

As used herein, the terms "CD276 antigen", "CD276", "4Ig-B7-H3", "B7 homolog 3", or "B7H3" are equivalent and refer to the product of the human B7H3 gene (Gene ID: 80381, UniGene Hs.744915). This protein may participate in the regulation of T-cell-mediated immune response (UniProt accession number: Q5ZPR3). The GenBank entry of the sequence of the mRNA of the human B7H3 protein is AF302102.1.

As used herein, the terms "JAM2" or "junctional adhesion molecule 2" or "CD322" are equivalent and refer to the product of the human JAM2 gene (Gene ID: (Human) 58494). This protein may play a role in the processes of lymphocyte homing to secondary lymphoid organs (UniProt ID: (Human) P57087). The GenBank entry of the sequence of the DNA sequence are for example NC_000021.8, NT_011512.12 and NC_018932.2.

As used herein, the term "antibody" refers to immunoglobulin molecules and immunologically active portions of immunoglobulin molecules, i.e., molecules that contain an antigen-binding site that immunospecifically binds an antigen. As such, the term antibody encompasses not only whole antibody molecules, but also antigen-binding antibody fragments as well as variants (including derivatives) of antibodies and antibody fragments. In particular, the antibody according to the disclosure may correspond to a monoclonal antibody (e.g. a chimeric, humanized or human antibody), or a fragment of monoclonal antibody. The term antibody refers to classical antibodies as well as to Heavy-chain antibodies and fragments and derivatives thereof such as (VHH)2 fragments and single domain antibodies.

As used herein, the term "classical antibody" refers to a large Y-shaped glycoprotein that is typically made of two large heavy-chains linked to each other by disulfide bonds, each heavy chain being linked to a small light-chain by a disulfide bond. Each chain is composed of structural domains, i.e. immunoglobulin domains. These domains contain about 70-110 amino acids and are classified into variable (IgV), and constant (IgC) domains. Antibodies are capable of recognizing a unique molecule, i.e. an antigen, an epitope or a ligand, via its variable regions located at the tip of the "Y" of a classical antibody. In placental mammals there are five classical antibody isotypes known as IgA, IgD, IgE, IgG, and IgM that are classified according to the type of their heavy chains denoted by the Greek letters: α, δ, ε, γ, and µ respectively. Classical antibodies can polymerized, in particular to form dimers or pentamers.

As used herein, the term "heavy chain" refers to a polypeptide constituted of two regions, the constant region formed of three or four immunoglobulin constant domains depending on the type of heavy chain and the variable region formed of a single immunoglobulin variable domain.

As used herein, the term "light chain" refers to a polypeptide constituted of two regions, the constant region formed of a single immunoglobulin constant domain and the variable region formed of a single immunoglobulin variable domain. In mammals there are two types of immunoglobulin light chain, which are called lambda (λ) and kappa (x).

As used herein the term "variable domain" refers to the immunoglobulin domain of a heavy or of a light chain that is responsible for binding to an antigen. A variable domain comprise several loops referred to as hypervariable or complementarity determining regions (CDRs) which are responsible for binding to the antigen.

As used herein "VH" refers to the variable domain of a heavy chain.

As used herein, the term "Fc (Fragment crystallizable) region" refers to the part of the heavy chain corresponding to the first two or three immunoglobulin constant domain (depending on the type of heavy chain) present at the base of the "Y" in a classical antibody. The Fc region contains a conserved glycosylation site involved in different interactions.

An "antibody fragment" of classical antibodies comprises a portion of an intact antibody, preferably the antigen binding or variable region of the intact antibody. Examples of antibody fragments include Fv, Fab, Fab', F(ab)2, F(ab')2, F(ab)3, Fv (typically the VL and VH domains of a single arm of an antibody), single-chain Fv (scFv), di-scFvs or sc(Fv)2, dsFv, Fd (typically the VH and CH1 domain), dAb (typically a VH domain), CDRs, VH, VL, minibodies, diabodies and multi-specific antibodies formed from antibodies fragments.

The term "Fab" denotes an antibody monovalent fragment having a molecular weight of about 50,000 and antigen binding activity, and consisting of the light and heavy chains variable domains (VL and VH), the light chain constant domain (CL) and the first heavy chain constant domain (CH1) domains which can be obtained by cutting a disulfide bond of the hinge region of the F(ab')2 fragment.

The term "Fv" refers to the N-terminal part of the Fab fragment and consists of the variable portions of a light chain and a heavy chain.

The term "F(ab')2" refers to an antibody bivalent fragment having a molecular weight of about 100,000 and antigen binding activity, which comprises two Fab fragments linked by a disulfide bridge at the hinge region.

The term "Fd" refers to an antibody fragment consisting of the VH and CH1 domains.

The term "dAb" (Ward et al., 1989 Nature 341:544-546) refers to a single variable domain antibody, i.e. an antibody fragment which consists of a VH or VL domain.

A single chain Fv ("scFv") polypeptide is a covalently linked VH::VL heterodimer which is usually expressed from a gene fusion including VH and VL encoding genes linked by a peptide-encoding linker. "dsFv" is a VH::VL heterodimer stabilized by a disulfide bond. Divalent and multivalent antibody fragments can form either spontaneously by association of monovalent scFvs such as di-scFvs, or can be generated by coupling monovalent scFvs by a peptide linker, such as divalent sc(Fv)2.

The term "diabodies" refers to small antibody fragments with two antigen-binding sites, which fragments comprise a VH domain connected to a VL domain in the same polypeptide chain (VH-VL). By using a linker that is too short to allow pairing between the two domains on the same chain, the domains are forced to pair with the complementarity domains of another chain and create two antigen-binding sites. The diabody may be mono- or bi-specific.

Antibody fragments which recognize specific epitopes can be generated by known techniques. The antibody fragments are antigen binding portions of an antibody, such as F(ab')2, Fab, Fv, scFv and the like. Other antibody fragments include, but are not limited to: the F(ab')2 fragments which can be produced by pepsin digestion of the antibody molecule and the Fab' fragments, which can be generated by reducing disulfide bridges of the F(ab')2 fragments. Alternatively, Fab' expression libraries can be constructed (Huse et al., 1989, Science, 246:1274-1281) to allow rapid and easy identification of monoclonal Fab' fragments with the desired specificity.

As used herein, the terms "Heavy-chain antibody" or "HCAbs" refer to immunoglobulins which are devoid of light chains and consist in two heavy chains. These antibodies do not rely upon the association of heavy and light chain variable domains for the formation of the antigen-binding site but instead the variable domain of the heavy polypeptide chains alone naturally forms the complete antigen binding site. Each heavy chain comprises a constant region and a variable domain which enables the binding to a specific antigen, epitope or ligand. As used herein, HCAbs encompass heavy chain antibodies of the camelid-type in which each heavy chain comprises a variable domain called VHH and two constant domains. Such heavy-chain antibodies directed against a specific antigen can be obtained from immunized camelids. Camelids encompass dromedary, camel, lama and alpaca. Camelid HCAbs have been described by Hamers-Casterman et al., Nature, 1993, 363:446. Other examples of HCAb are immunoglobulin-like structures (Ig-NAR) from cartilaginous fishes. Heavy-chain antibodies can be humanized using well-known methods.

The terms "single domain antibody", "sdAb" and "nanobody" are used interchangeably and have the same meaning. As used herein, the term single domain antibody refers to a single variable domain derived from a heavy chain antibody, which is able to bind an antigen, an epitope or a ligand alone, that is to say, without the requirement of another binding domain. A single domain antibody may be or may derive from VHH and V-NAR. V-NAR refers to the variable domain found in immunoglobulin-like structures (Ig-NAR) discovered in cartilaginous fishes such as sharks. As an alternative, single domain antibody may be obtained from human VH by camelization, in particular with F37, E44, R45 and F47 mutations. For review about single domain antibodies, one may refer to Saerens et al., Current Opinion in Pharmacology, 2008, 8:600-608. In a preferred aspect, the single domain antibody according to the disclosure is a synthetic single domain antibody.

As used herein, the term "synthetic" means that such antibody has not been obtained from fragments of naturally occurring antibodies but produced from recombinant nucleic acids comprising artificial coding sequences (cf. WO 2015/063331).

The term "VHH", as used herein, refers to an antibody fragment consisting of the VH domain of camelid heavy-chain antibody. VHH fragments can be produced through recombinant DNA technology in a number of microbial hosts (bacterial, yeast, mould), as described in WO 94/29457. Altematively, binding domains can be obtained by modification of the VH fragments of classical antibodies by a procedure termed "camelization", described by Davies et al, 1995. Dimers of VHH fragments, i.e. (VHH)2, can be generated by fusing two sequences encoding VHH fragments, end to end, e.g. by PCR. Preferably, the (VHH)2 fragment is monospecific. The two VHH of a (VHH)2 may also recognize two different antigen, i.e. the (VHH)2 may be bispecific.

The variable domain of an antibody of the disclosure comprises at least three complementarity determining region (CDR) which determines its binding specificity. Preferably, in a variable domain, the CDRs are distributed between framework regions (FRs). The variable domain thus contains at least 4 framework regions interspaced by 3 CDR regions, resulting in the following typical antibody variable domain structure: FR1-CDR1-FR2-CDR2-FR3-CDR3-FR4. CDRs and/or FRs of the single domain antibody of the disclosure may be fragments or derivatives from a naturally-occurring antibody variable domain or may be synthetic.

A "humanized antibody" is a chimeric, genetically engineered, antibody in which the CDRs from an antibody, e.g. a mouse antibody (donor antibody), are grafted onto a human antibody (acceptor antibody). Thus, a humanized antibody is an antibody having CDRs from a donor antibody and variable region framework and constant regions, when present, from a human antibody.

Likewise, a "camelized antibody" is an antibody having CDRs from a donor antibody, preferably a human donor, and variable region framework and constant regions, when present, from a Camelid antibody.

The term "monoclonal antibody" or "mAb" as used herein refers to an antibody molecule of a single amino acid composition, that is directed against a specific antigen and which may be produced by a single clone of B cells or hybridoma, or by recombinant methods. The use of antibody components derived from humanized monoclonal antibodies obviates potential problems associated with the immunogenicity of murine constant regions. Rodent monoclonal antibodies to specific antigens may be obtained by methods known to those skilled in the art. (See, e.g., Kohler and Milstein, Nature 256: 495 (1975), and Coligan et al. (eds.), CURRENT PROTOCOLS IN IMMUNOLOGY, VOL. 1, pages 2.5.1-2.6.7 (John Wiley & Sons 1991)).

Antibodies according to the disclosure may be produced by any technique known in the art, such as, without limitation, any chemical, biological, genetic or enzymatic technique, either alone or in combination. The antibodies of the disclosure can be obtained by producing and culturing hybridomas. See also WO 2015/063331 for the production of synthetic single domain antibodies.

The terms "kit", "product" or "combined preparation", as used herein, defines especially a "kit of parts" in the sense that the combination partners (a) and (b), as defined in the present application can be dosed independently or by use of different fixed combinations with distinguished amounts of the combination partners (a) and (b), i.e. simultaneously or at different time points. The parts of the kit of parts can then be administered simultaneously or chronologically staggered, that is at different time points for any part of the kit of parts. The ratio of the total amounts of the combination partner (a) to the combination partner (b) to be administered in the combined preparation can be varied. The combination partners (a) and (b) can be administered by the same route or by different routes.

As used herein, the term "simultaneous" refers to a pharmaceutical composition, a kit, a product or a combined preparation according to the disclosure in which the active ingredients are used or administered simultaneously, i.e. at the same time.

As used herein, the term "sequential" refers to a pharmaceutical composition, a kit, a product or a combined preparation according to the disclosure in which the active ingredients are used or administered sequentially, i.e. one after the other. Preferably, when the administration is sequential, all the active ingredients are administered in less than about an hour, preferably less than about 10 minutes, even more preferably in less than about a minute.

As used herein, the term "separate" refers to a pharmaceutical composition, a kit, a product or a combined preparation according to the disclosure in which the active ingredients are used or administered at distinct time of the day. Preferably, when the administration is separate, the active ingredients are administered with an interval of about 1 hour to about 24 hours, preferably with an interval of about 1 hour and 15 hours, more preferably with an interval of about 1 hour and 8 hours, even more preferably with an interval of about 1 hour and 4 hours.

The methods of the invention as disclosed below, may be *ex vivo* or *in vitro* methods, preferably *in vitro* methods.

In the present application, any aspect disclosed in the present application and comprising the list "DPP4, and/or CD73, and/or B7H3, and/or OX40L, and/or JAM2, and/or PDL2" can be read with any one of the following combinations:
- DPP4;
- CD73;
- B7H3;
- OX40L;
- JAM2;
- PDL2;
- DPP4 and CD73;
- DPP4 and B7H3;
- DPP4 and OX40L;
- DPP4 and JAM2;
- DPP4 and PDL2;
- CD73 and B7H3;
- CD73 and OX40L;
- CD73 and JAM2;
- CD73 and PDL2;
- B7H3 and OX40L;
- B7H3 and JAM2;
- B7H3 and PDL2;
- OX40L and JAM2;
- OX40L and PDL2;
- JAM2 and PDL2;
- DPP4 and CD73 and B7H3;
- DPP4 and CD73 and OX40L;
- DPP4 and CD73 and JAM2;
- DPP4 and CD73 and PDL2;
- DPP4 and B7H3 and OX40L;
- DPP4 and B7H3 and JAM2;
- DPP4 and B7H3 and PDL2;
- DPP4 and OX40L and JAM2;
- DPP4 and OX40L and PDL2;
- DPP4 and JAM2 and PDL2;
- CD73 and B7H3 and OX40L;
- CD73 and B7H3 and JAM2;
- CD73 and B7H3 and PDL2;
- CD73 and OX40L and JAM2;
- CD73 and OX40L and PDL2;
- CD73 and JAM2 and PDL2;
- B7H3 and OX40L and JAM2;
- B7H3 and OX40L and PDL2;
- B7H3 and JAM2 and PDL2;
- OX40L and JAM2 and PDL2;
- DPP4 and CD73 and B7H3 and OX40L;
- DPP4 and CD73 and B7H3 and JAM2;
- DPP4 and CD73 and B7H3 and PDL2;
- DPP4 and CD73 and OX40L and JAM2;
- DPP4 and CD73 and OX40L and PDL2;
- DPP4 and CD73 and JAM2 and PDL2;
- DPP4 and B7H3 and OX40L and JAM2;
- DPP4 and B7H3 and OX40L and PDL2;
- DPP4 and B7H3 and JAM2 and PDL2;
- DPP4 and OX40L and JAM2 and PDL2;
- CD73 and B7H3 and OX40L and JAM2;
- CD73 and B7H3 and OX40L and PDL2;
- CD73 and B7H3 and JAM2 and PDL2;
- B7H3 and OX40L and JAM2 and PDL2;
- DPP4 and CD73 and B7H3 and OX40L and JAM2;
- DPP4 and CD73 and B7H3 and OX40L and PDL2;
- DPP4 and CD73 and B7H3 and JAM2 and PDL2;
- DPP4 and B7H3 and OX40L and JAM2 and PDL2;
- CD73 and B7H3 and OX40L and JAM2 and PDL2;
- DPP4 and CD73 and B7H3 and OX40L and JAM2 and PDL2.

In the present application, any aspect disclosed in the present application and comprising the list "DPP4, and/or CD73, and/or B7H3, and/or OX40L, and/or PDL2" can be read with any one of the following combinations:
- DPP4;
- CD73;
- B7H3;
- OX40L;
- PDL2;
- DPP4 and CD73;
- DPP4 and B7H3;
- DPP4 and OX40L;
- DPP4 and PDL2;
- CD73 and B7H3;
- CD73 and OX40L;
- CD73 and PDL2;
- B7H3 and OX40L;
- B7H3 and PDL2;
- OX40L and PDL2;
- DPP4 and CD73 and B7H3;
- DPP4 and CD73 and OX40L;
- DPP4 and CD73 and PDL2;
- DPP4 and B7H3 and OX40L;
- DPP4 and B7H3 and PDL2;
- DPP4 and OX40L and PDL2;
- CD73 and B7H3 and OX40L;
- CD73 and B7H3 and PDL2;
- CD73 and OX40L and PDL2;
- B7H3 and OX40L and PDL2;
- DPP4 and CD73 and B7H3 and OX40L;
- DPP4 and CD73 and B7H3 and PDL2;
- DPP4 and CD73 and OX40L and PDL2;
- DPP4 and B7H3 and OX40L and PDL2;
- CD73 and B7H3 and OX40L and PDL2;
- DPP4 and CD73 and B7H3 and OX40L and PDL2;

In the present application, any aspect disclosed in the present application and comprising the list "DPP4, and/or CD73, and/or B7H3, and/or OX40L, and/or JAM2" can be read with any one of the following combinations:
- DPP4;
- CD73;
- B7H3;
- OX40L;
- JAM2;
- DPP4 and CD73;
- DPP4 and B7H3;
- DPP4 and OX40L;
- DPP4 and JAM2;
- CD73 and B7H3;
- CD73 and OX40L;
- CD73 and JAM2;
- B7H3 and OX40L;
- B7H3 and JAM2;
- OX40L and JAM2;
- DPP4 and CD73 and B7H3;
- DPP4 and CD73 and OX40L;
- DPP4 and CD73 and JAM2;
- DPP4 and B7H3 and OX40L;
- DPP4 and B7H3 and JAM2;
- DPP4 and OX40L and JAM2;
- CD73 and B7H3 and OX40L;
- CD73 and B7H3 and JAM2;
- CD73 and OX40L and JAM2;
- B7H3 and OX40L and JAM2;
- DPP4 and CD73 and B7H3 and OX40L;
- DPP4 and CD73 and B7H3 and JAM2;
- DPP4 and CD73 and OX40L and JAM2;
- DPP4 and B7H3 and OX40L and JAM2;
- CD73 and B7H3 and OX40L and JAM2;
- DPP4 and CD73 and B7H3 and OX40L and JAM2;

It will be understood that these lists refer to any aspect related to the above cited marker(s), such as but not limited to CAFs population positive for such marker(s), CAFs level associated with such marker(s), percentage of cells or CAFs associated with such marker(s), or expression level of such marker(s).

In the first aspect which is not claimed, the present disclosure concerns a method for selecting a patient affected with a tumor for an immunotherapy treatment or for predicting the response of a subject affected with a tumor to an immunotherapy treatment, wherein the method comprises:
(a) detecting DPP4⁺ CAFs (Cancer Associated Fibroblast), and/or CD73⁺ CAFs, and/or B7H3⁺ CAFs, and/or OX40L⁺ CAFs, and/or JAM2⁺ CAFs, and/or PDL2⁺ CAFs in a cancer sample from said patient;
(b) determining the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample, wherein the responsiveness of said patient to an immunotherapy treatment is inversely proportional to the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in the cancer sample;
(c) optionally, selecting patients with low level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs as suitable for an immunotherapy treatment, and selecting patients with high level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs for a cancer treatment excluding immunotherapy.

Optionally, the method may further comprise a step of providing a cancer sample from said patient before the step (a).

In a particular aspect, the patients are selected at step (c) as suitable for an immunotherapy treatment when two, preferably three, more preferably four, even more preferably five of the levels selected from the group constituted of DPP4⁺ CAFs level, OX40L⁺ CAFs level, CD73⁺ CAFs level, JAM2⁺ CAFs level, PDL2⁺ CAFs level, and B7H3⁺ CAFs level, are low.

In another particular aspect, the patients are selected at step c) for a cancer treatment, at the exclusion of an immunotherapy, when two, preferably three, more preferably four, even more preferably five of the levels selected from the group constituted of DPP4⁺ CAFs level, OX40L⁺ CAFs level, CD73⁺ CAFs level, JAM2⁺ CAFs level, PDL2⁺ CAFs level, and B7H3⁺ CAFs level, are high.

The cancer treatment may be selected from the group consisting of surgery, chemotherapy, radiation therapy, hormonal therapy, targeted therapy and palliative care, or a combination thereof.

### Detection of CAF subpopulations

The method disclosed but not claimed herein comprises a step of detecting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient.

As used herein, the term "DPP4⁺ CAFs" or "DPP4⁺ Cancer Associated Fibroblast" refers to a subpopulation of CAFs that express DPP4. Other CAFs do not express DPP4. Therefore, in a preferred embodiment, the detection of DPP4⁺ CAFs relies on the detection of CAFs expressing DPP4. However, other markers specific of DPP4⁺ CAFs could also be used to detect them.

As used herein, the term "OX40L⁺ CAFs" or "OX40L⁺ Cancer Associated Fibroblast" refers to a subpopulation of CAFs that express OX40L. Other CAFs do not express OX40L. Therefore, in a preferred embodiment, the detection of OX40L⁺ CAFs relies on the detection of CAFs expressing OX40L. However, other markers specific of OX40L⁺ CAFs could also be used to detect them.

As used herein, the term "CD73⁺ CAFs" or "CD73⁺ Cancer Associated Fibroblast" refers to a subpopulation of CAFs that express CD73. Other CAFs do not express CD73. Therefore, in a preferred embodiment, the detection of CD73⁺ CAFs relies on the detection of CAFs expressing CD73. However, other markers specific of CD73⁺ CAFs could also be used to detect them.

As used herein, the term "PDL2⁺ CAFs" or "PDL2⁺ Cancer Associated Fibroblast" refers to a subpopulation of CAFs that express PDL2. Other CAFs do not express PDL2. Therefore, in a preferred embodiment, the detection of PDL2⁺ CAFs relies on the detection of CAFs expressing PDL2. However, other markers specific of PDL2⁺ CAFs could also be used to detect them.

As used herein, the term "B7H3⁺ CAFs" or "B7H3⁺ Cancer Associated Fibroblast" refers to a subpopulation of CAFs that express B7H3. Other CAFs do not express B7H3. Therefore, in a preferred embodiment, the detection of B7H3⁺ CAFs relies on the detection of CAFs expressing B7H3. However, other markers specific of B7H3⁺ CAFs could also be used to detect them.

As used herein, the term "JAM2⁺ CAFs" or "JAM⁺ Cancer Associated Fibroblast" refers to a subpopulation of CAFs that express JAM2. Other CAFs do not express JAM2. Therefore, in a preferred embodiment, the detection of JAM2⁺ CAFs relies on the detection of CAFs expressing JAM2. However, other markers specific of JAM2⁺ CAFs could also be used to detect them.

A CAF is considered as expressing DPP4, OX40L, CD73, PDL2, JAM2 or B7H3 when the level of the mRNA thereof, and/or the level of the protein thereof, and/or the level of the activity of the protein thereof, for example the level of an enzymatic activity, is significantly different from the level of the corresponding background noise, for example the level measured in the same conditions but in the absence of cells, and/or from the level of the corresponding control condition, for example the level measured in the same conditions but with cells known for not expressing DPP4, OX40L, CD73, PDL2, JAM2 or B7H3 respectively.

A CAF is considered as not expressing DPP4, OX40L, CD73, PDL2, JAM 2 or B7H3 when the level of the mRNA thereof, and/or the level of the protein thereof, and/or the level of the activity of the protein thereof, for example the level of an enzymatic activity, is not significantly different from the level of the corresponding background noise, for example the level measured in the same conditions but in the absence of cells, and/or from the level of the corresponding control condition, for example the level measured in the same conditions but with cells known for not expressing DPP4, OX40L, CD73, PDL2, JAM2 or B7H3.

The measurement of DPP4, OX40L, CD73, PDL2, JAM2 or B7H3 expression level in a CAF can be implemented by a variety of techniques well known by the skilled person. In particular, the measure of the expression level of DPP4, OX40L, CD73, PDL2, JAM2 or B7H3 in CAFs can rely on the detection of DPP4, OX40L, CD73, PDL2, JAM2 or B7H3 mRNA, protein or protein activity, in particular enzymatic activity.

In one aspect, the expression level of DPP4, OX40L, CD73, PDL2, JAM2 or B7H3 in a CAF is determined by measuring the expression of their mRNA. Methods for determining the quantity of mRNA in a cell are well known by the man skilled in the art. mRNA can be detected by hybridization (e. g., Northern blot analysis) in particular by the Nanostring method and/or by amplification (e.g., RT-PCR), in particular by quantitative or semi-quantitative RT-PCR. Other methods of Amplification include ligase chain reaction (LCR), transcription-mediated amplification (TMA), strand displacement amplification (SDA) and nucleic acid sequence based amplification (NASBA).

Real-time quantitative or semi-quantitative RT-PCR is particularly advantageous. Taqman probes specific of the protein of interest transcript may be used. In a preferred embodiment, the expression level of DPP4, OX40L, CD73, PDL2, JAM2, B7H3 and any other protein of interest is determined by measuring the quantity of their mRNA, preferably by quantitative or semi-quantitative RT-PCR or by real-time quantitative or semi-quantitative RT-PCR.

As used herein, the terms "quantitative RT-PCR", "qRT-PCR", "Real time RT-PCR" and "quantitative Real time RT-PCR" are equivalent and can be used interchangeably. Any of a variety of published quantitative RT-PCR protocols can be used (and modified as needed) for use in the present method. Suitable quantitative RT-PCR procedures include but are not limited to those presented in U.S. Pat. No. 5,618,703 and in U.S. Patent Application No. 2005/0048542.

In a preferred embodiment of the above mentioned method, the quantitative RT-PCR includes two main steps, the reverse transcription (RT) of RNA in cDNA and the quantitative PCR (Polymerase Chain Reaction) amplification of the cDNA. Quantitative RT-PCR can be performed by an uncoupled or by a coupled procedure. In an uncoupled quantitative RT-PCR, the reverse transcription is performed independently from the quantitative PCR amplification, in separate reactions. Whereas, in a coupled quantitative RT-PCR, the reverse transcription and the quantitative PCR amplification are performed in a single reaction tube using a common reaction mixture including both the reverse transcriptase and the DNA polymerase. The method disclosed herein encompasses all versions of quantitative RT-PCR.

Quantitative RT-PCR Primers that can be used to measure DPP4 expression level in a CAF are for example: Forward: 5'-AGTGGCGTGTTCAAGTGTGG-3' (SEQ ID NO: 1) and Reverse: 5'-CAAGGTTGTCTTCTGGAGTTGG-3 (SEQ ID NO: 2).

Quantitative RT-PCR Primers that can be used to measure OX40L expression level in a CAF are for example: Forward: 5'-CCTCGAATTCAAAGTATCAAAG-3' (SEQ ID NO: 3) and Reverse: 5'-GTGAGGATGAAACCTTTCTCC-3' (SEQ ID NO: 4).

Quantitative RT-PCR Primers that can be used to measure CD73 expression level in a CAF are for example: Forward: 5'-CTCCTCTCAATCATGCCGCT-3' (SEQ ID NO: 5) and Reverse: 5'-TGGATTCCATTGTTGCGTTCA-3' (SEQ ID NO: 6).

Quantitative RT-PCR Primers that can be used to measure PDL2 expression level in a CAF are for example: Forward: 5'-ACAGTGCTATCTGAACCTGTG-3' (SEQ ID NO: 7) and Reverse: 5'-GTCATATCAGGTCACCCTGGC-3' (SEQ ID NO: 8).

Quantitative RT-PCR Primers that can be used to measure B7H3 expression level in a CAF are for example: Forward: 5'-CTGGCTTTCGTGTGCTGGAGAA-3' (SEQ ID NO: 9) and Reverse: 5'-GCTGTCAGAGTGTTTCAGAGGC-3' (SEQ ID NO: 10).

Quantitative RT-PCR Primers that can be used to measure JAM2 expression level in a CAF are for example: Forward: 5'- CGCCCTGGGCTATCATAAGG -3' (SEQ ID NO: 11) and Reverse: 5'- CAAAGGAGACACTCCGACCC -3' (SEQ ID NO: 12).

In a most preferred aspect, the expression level of DPP4, OX40L, CD73, PDL2, JAM2 or B7H3 in a CAF is determined by measuring the expression of their mRNA by a single cell RNA-Seq method as described in the Experimental part.

The Nanostring method is also very advantageous. It is a hybridization method that allows to quantify RNA without requiring linear nor exponential amplification. It is a very sensitive method, since only 10 ng of mRNA are needed to perform it, allowing analysis of quantity limited biological samples. The restricted number of sample manipulation steps together with the absence of enzymatic reaction allows precise and physiologically correct quantifications. This method is also extremely flexible since it can be applied to various types of samples. In a preferred embodiment, the expression level of DPP4, OX40L, CD73, PDL2, JAM2, B7H3 and any other protein of interest is determined by measuring the quantity of their mRNA, preferably by the Nanostring method.

The Nanostring method necessitates the use of a pair of probes specifically designed for each mRNA. The first probe, called the capture-probe, specifically hybridizes the target mRNA and binds it to a solid support, preferably a counting stand. Preferably, the capture probe is linked to a molecule that allows the probe to bind the solid support, more preferably said molecule is biotin, thereby immobilizing the mRNA of interest onto the counting stand. The second probe, called the reporter-probe, specifically hybridizes the mRNA of interest and is linked to a label that allows the detection and quantification of the mRNA. Preferably, this label is a fluorescent label. More preferably the label is made of a combination of fluorochromes. Even more preferably the label is made of a combination of 6 fluorochromes chosen among 4 fluorochromes of different colors, defining a code specific to each target mRNA. This color code confers to the technique a very high sensitivity and enables the analysis of quantity-limited biological samples. When several reporter probes are used simultaneously to determine the amounts of several mRNA, each reporter probe is linked to a different label, preferably a different combination of 6 fluorochromes chosen among 4 fluorochromes of different colors.

Preferably, the fluorescence is analyzed by an nCounter, an optical system that is capable to identify the color codes.

Nanostring probes such as NM_001935.3:2700 (NanoString Technologies) can be used to measure DPP4 expression level in a CAF.

Nanostring probes such as NM_003326.2:545 (NanoString Technologies) can be used to measure OX40L expression level in a CAF.

Nanostring probes such as NM_002526.2:1214 (NanoString Technologies) can be used to measure CD73 expression level in a CAF.

Nanostring probes such as NM_025239.3:235 (NanoString Technologies) can be used to measure PD-L2 expression level in a CAF.

Nanostring probes such as NM_001024736.1:2120 (NanoString Technologies) can be used to measure B7H3 expression level in a CAF.

Nanostring probes such as NM_001270407.1 (NanoString Technologies) can be used to measure JAM2 expression level in a CAF.

In a preferred aspect, the expression level of DPP4, OX40L, CD73, PDL2, JAM2 or B7H3 in a CAF is determined by measuring the expression of their respective proteins.

The quantity of a protein may be measured by any method known by the skilled person. Usually, these methods comprise contacting the sample with a binding partner capable of selectively interacting with the protein present in the sample. The binding partner is generally a polyclonal or monoclonal antibody, preferably a monoclonal antibody. Such an antibody can be produced through methods known to the man skilled in the art. This antibody includes in particular those produced by a hybridoma and those produced by genetic engineering using host cells transformed with a recombinant expression vector carrying a gene encoding the antibody. A hybridoma producing monoclonal antibodies can be obtained as following: the protein or immunogenic fragments thereof are used as antigens for immunization according to conventional methods of immunization. The resulting immunocytes are fused with known parent cells according to conventional cell fusion methods and the cells producing the antibodies are thus screened from fused cells using conventional screening methods.

The antibody according to the disclosure (which is not claimed) can be labelled and/or fused to a detection entity. Preferably, the antibody according to the disclosure is labelled or fused to a detection entity.

In a preferred aspect, the antibody is labelled. The antibody can be labelled with a label selected from the group consisting in a radiolabel, an enzyme label, a fluorescent label, a biotin-avidin label, a chemiluminescent label, and the like. The antibody according to the disclosure can be labeled by standard labeling techniques well known by the man skilled in the art and labelled antibodies can be visualized using known methods. In particular, labels generally provide signals detectable by fluorescence, chemiluminescence, radioactivity, colorimetry, mass spectrometry, X-ray diffraction or absorption, magnetism, enzymatic activity, or the like.

Preferably, the detectable label may be a luminescent label. For example, fluorescent labels, bioluminescent labels, chemiluminescent labels, and colorimetric labels may be used in the practice of the disclosure, more preferably a fluorescent label. Preferably, the label is linked at the C-terminal extremity of the antibody.

In another preferred aspect, the above mentioned antibody can be fused to a detection entity. The detection entity may be selected from the group consisting of a tag, an enzyme or a fluorescent protein. Preferably, the detection entity is at the C-terminal extremity of the antibody.

The disclosure concems antibodies specific of human DPP4, OX40L, CD73, PDL2, JAM2 or B7H3.

The quantity of DPP4, OX40L, CD73, PDL2, JAM2 or B7H3 may be measured by semi-quantitative Western blots, enzyme-labeled and mediated immunoassays, such as ELISAs, biotin/avidin type assays, radioimmunoassay, immunohistochemistry, immunoelectrophoresis or immunoprecipitation, protein or antibody arrays, or flow cytometry, such as Fluorescence-activated cell sorting (FACS). The reactions generally include revealing labels such as fluorescent, chemiluminescent, radioactive, enzymatic labels or dye molecules, or other methods for detecting the formation of a complex between the antigen and the antibody or antibodies reacted therewith. Preferably, the protein expression level is assessed by FACS or by immunohistochemistry.

Fluorescence-activated cell sorting (FACS) is a specialized type of flow cytometry. It provides a method for sorting a heterogeneous mixture of biological cells into two or more containers, one cell at a time, based upon the specific light scattering and fluorescent characteristics of each cell. The cell suspension is entrained in the center of a narrow, rapidly flowing stream of liquid. The flow is arranged so that there is a large separation between cells relative to their diameter. A vibrating mechanism causes the stream of cells to break into individual droplets. The system is adjusted so that there is a low probability of more than one cell per droplet. Just before the stream breaks into droplets, the flow passes through a fluorescence measuring station where the fluorescent character of each cell of interest is measured. An electrical charging ring is placed just at the point where the stream breaks into droplets. A charge is placed on the ring based immediately prior to fluorescence intensity being measured, and the opposite charge is trapped on the droplet as it breaks from the stream. The charged droplets then fall through an electrostatic deflection system that diverts droplets into containers based upon their charge.

Immunohistochemistry (IHC) refers to the process of selectively imaging antigens (e.g. proteins) in cells of a tissue section by exploiting the principle of antibodies binding specifically to antigens in biological tissues. Visualizing the antibody-antigen interaction can be accomplished in a number of ways, well known by the man skilled in the art. In the most common instance, an antibody is conjugated to an enzyme, such as peroxidase, that can catalyze a color-producing reaction or is tagged by a fluorophore, such as fluorescein or rhodamine. Immunohistochemistry can be divided into two phases: sample preparation and sample labeling.

Preparation of the sample is critical to maintain cell morphology, tissue architecture and the antigenicity of target epitopes. This requires proper tissue collection, fixation and sectioning. A solution of paraformaldehyde is often used to fix tissue, but other methods may be used. The tissue may then be sliced or used whole, dependent upon the purpose of the experiment or the tissue itself. Before sectioning, the tissue sample may be embedded in a medium, like paraffin wax or cryomedia. Sections can be sliced on a variety of instruments, most commonly a microtome, cryostat, or Compresstome tissue slicer. Specimens are typically sliced at a range of 3 µm-50 µm. The slices are then mounted on slides, dehydrated using alcohol washes of increasing concentrations (e.g., 50%, 75%, 90%, 95%, 100%), and cleared using a detergent like xylene before being imaged under a microscope. Depending on the method of fixation and tissue preservation, the sample may require additional steps to make the epitopes available for antibody binding, including deparaffinization and antigen retrieval. For formalin-fixed paraffin-embedded tissues, antigen-retrieval is often necessary, and involves pre-treating the sections with heat or protease. These steps may make the difference between the target antigens staining or not staining. Depending on the tissue type and the method of antigen detection, endogenous biotin or enzymes may need to be blocked or quenched, respectively, prior to antibody staining. Although antibodies show preferential avidity for specific epitopes, they may partially or weakly bind to sites on nonspecific proteins (also called reactive sites) that are similar to the cognate binding sites on the target antigen. To reduce background staining in **IHC,** samples are incubated with a buffer that blocks the reactive sites to which the primary or secondary antibodies may otherwise bind. Common blocking buffers include normal serum, non-fat dry milk, BSA, or gelatin. Methods to eliminate background staining include dilution of the primary or secondary antibodies, changing the time or temperature of incubation, and using a different detection system or different primary antibody. Quality control should as a minimum include a tissue known to express the antigen as a positive control, and negative controls of tissue known not to express the antigen, as well as the test tissue probed in the same way with omission of the primary antibody (or better, absorption of the primary antibody).

For immunohistochemical detection strategies, antibodies are classified, when necessary, as primary or secondary reagents. Primary antibodies are raised against an antigen of interest and are typically unconjugated (i.e. unlabeled), while secondary antibodies are raised against immunoglobulins of the primary antibody species. The secondary antibody is usually labelled and/or fused to a detection entity as described above.

The direct method is a one-step staining method and involves a labeled antibody reacting directly with the antigen in tissue sections. While this technique utilizes only one antibody and therefore is simple and rapid, the sensitivity is lower due to little signal amplification, in contrast to indirect approaches.

The indirect method involves an unlabeled primary antibody (first layer) that binds to the target antigen in the tissue and a labeled secondary antibody (second layer) that reacts with the primary antibody. The secondary antibody must be raised against the IgG of the animal species in which the primary antibody has been raised. This method is more sensitive than direct detection strategies because of signal amplification due to the binding of several secondary antibodies to each primary antibody if the secondary antibody is conjugated to the fluorescent or enzyme reporter. Further amplification can be achieved if the secondary antibody is conjugated to several biotin molecules, which can recruit complexes of avidin-, streptavidin- or NeutrAvidin protein-bound enzyme.

Preferably, the protein expression level is assessed by FACS or by immunohistochemistry as described in the experimental part.

Antibodies that can be used to measure DPP4 expression level in a CAF by FACS or immunohistochemistry are for example: the anti-human DPPIV/CD26 antibody of reference AF1180 (from R&D systems), or the PE mouse anti-Human CD26, clone M-A261, of reference 555437 (from BD Biosciences).

Antibodies that can be used to measure OX40L expression level in a CAF are for example: the anti-TNFSF4 antibody of reference HPA059579 (from Sigma), the BV421 Mouse anti-Human OX40 Ligand, clone ik-1, of reference 563766 (BD Biosciences).

Antibodies that can be used to measure CD73 expression level in a CAF are for example: the BUV737 Mouse anti-human CD73, clone AD2, of reference 565395 (from BD Biosciences).

Antibodies that can be used to measure JAM2 expression level in a CAF are for example: the anti-JAM2 antibody of reference PA5-21576 (from ThermoFisher), of reference aa262-274 (from LifeSpan Biosciences), of reference OAAB10508 (from Aviva Systems Biology),

In a preferred aspect, the detection of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs by FACS in a cancer sample of a patient may comprise the following steps:
- exclusion of the non-CAF cells, for example by excluding the CD45⁺ cells, the EpCAM⁺ cells and the CD31⁺ cells and thereby selecting the CD45⁻EpCAM⁻CD31⁻cells; and/or
- selection of the CAF cells, for example by selecting the CD29⁺ cells and/or the PDGFRb⁺ cells, preferably by selecting the CD29⁺ cells; and
- optionally, exclusion of the dead cells, for example by using an intracellular dye such as the violet LIVE/DEAD dye or DAPI and excluding the stained cells; and
- detection of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in the cells obtained at the precedent step.

An anti-CD45 antibody that can be used is, for example, an anti-CD45-APC-Cy7 (BD Biosciences, #BD-557833).

An anti-EpCAM antibody that can be used is, for example, an anti-EpCAM-PerCR/Cy5.5 (BioLegend, #324214).

An anti-CD31 antibody that can be used is, for example, an anti-CD31-PECY7 (BioLegend, #303118).

An anti-CD29 antibody that can be used is, for example, an anti-CD29-Alexa Fluor 700 (BioLegend, #303020).

An anti-PDGFRb antibody that can be used is, for example, an anti-PDGFRb-PE (BioLegend, # 400114).

In another preferred aspect, the detection of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs by immunohistochemistry in a cancer sample of a patient may comprise the following steps:
- identification of the CAFs in the tissue section, for example on the basis of morphological criteria;
- detection of the DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs among the CAFs, based on DPP4, and/or OX40L, and/or CD73, and/or PDL2, and/or B7H3 antibodies immunostaining.

In another aspect, the expression level of DPP4 or CD73 in a CAF is determined by measuring the activity of the protein, in particular, the enzymatic activity of DPP4 or CD73. The enzymatic activity of a protein may be measured by any method known by the skilled person. Usually, enzyme assays measure either the consumption of substrate or production of product over time. A large number of different methods of measuring the concentrations of substrates and products exist such as:
- initial rate experiments in which an enzyme is mixed with a large excess of the substrate. The enzyme-substrate intermediate builds up fast and the reaction achieves a steady-state kinetics in which enzyme substrate intermediates remains approximately constant over time and the reaction rate changes relatively slowly. Rates are measured for a short period after the attainment of the quasi-steady state, typically by monitoring the accumulation of product with time. Because the measurements are carried out for a very short period and because of the large excess of substrate, the approximation that the amount of free substrate is approximately equal to the amount of the initial substrate can be made.

- progress curve experiments in which the kinetic parameters are determined from expressions for the species concentrations as a function of time. The concentration of the substrate or product is recorded in time after the initial fast transient and for a sufficiently long period to allow the reaction to approach equilibrium.
- transient kinetics experiments, in which reaction behavior is tracked during the initial fast transient as the intermediate reaches the steady-state kinetics period.
- relaxation experiments in which an equilibrium mixture of enzyme, substrate and product is perturbed, for instance by a temperature, pressure or pH jump, and the return to equilibrium is monitored. The analysis of these experiments requires consideration of the fully reversible reaction.

According to the nature of the substrate used, measurement of the substrate consumption or product production can be performed for example by spectrophotometric assays, for example colorimetric assays, fluorometric assays, calorimetric assays, chemiluminescent assays, light scattering assays, microscale thermophoresis assays, radiometric assays, or chromatographic assays. Preferably, measurement of the substrate consumption or product production is performed by a fluorometric assay.

DPP4 is a serine exopeptidase that catalyzes the release of an N-terminal dipeptide provided that the next to last residue is proline, hydroxyproline, dehydroproline or alanine. Only oligopeptides in the *trans* conformation are able to bind to the active site of DPP4. To assay DPP4 enzymatic activity, a non-fluorescent substrate from which a fluorescent product will be released upon DPP4 cleavage can be used. Such a substrate can be, for example, H-Gly-Pro-AMC, with AMC (7-Amino-4-Methyl Coumarin) as the released fluorescent product. DPP4 enzymatic activity can also be assayed by immunohistochemistry with Gly-Pro-4-methoxy-β-naphtylamide as a substrate (cf. Lojda Z et al, J Histochem Cytochem, 29: 481-493, 1981).

CD73 is an ecto-5'-nucleotidase that degrades AMP (Adenosine Mono Phosphate) into Adenosine and a free phosphate. A colorimetric detection kit can be used to measure the activity of the CD73 enzyme, such as the #k992-100 Biovision kit.

### CAF subpopulation levels

The method disclosed but not claimed herein comprises a step of determining the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient.

The level of a cell population in a sample can be for example a quantity of cells in this sample or any fraction of it, a ratio of the number of cells of this population on the total number of cells of the sample or any fraction of it, or the percentage of cells of this population on the total number of cells of the sample or any fraction of it.

Preferably, the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient are the percentage(s) of these cells on the total number of cells of the cancer sample.

Alternatively, the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs, in a cancer sample of a patient are the percentage(s) of these cells on the total number of stromal cells in the cancer sample.

Alternatively, the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient are the percentage(s) of these cells on the total number of CAFs in the cancer sample.

The present disclosure thus concems a method (which is not claimed) for selecting a patient affected with a tumor for an immunotherapy treatment or for predicting the response of a subject affected with a tumor to an immunotherapy treatment, wherein the method comprises:
(a) detecting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient;
(b) determining the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample, wherein the responsiveness of said patient to an immunotherapy treatment is inversely proportional to the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in the cancer sample;
(c) optionally, selecting patients with low percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs , in particular patients without any DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs, as suitable for an immunotherapy treatment and selecting patients with high percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs for a cancer treatment excluding immunotherapy.

Optionally, the method may further comprise a step of providing a cancer sample from said patient before the step (a).

In a particular aspect, the patients are selected at step (c) as suitable for an immunotherapy treatment when two, preferably three, more preferably four, even more preferably five of the percentages selected from the group constituted of DPP4⁺ CAFs percentage, OX40L⁺ CAFs percentage, CD73⁺ CAFs percentage, PDL2⁺ CAFs percentage, JAM2⁺ CAFs percentage and B7H3⁺ CAFs percentage, are low.

The cancer treatment may be selected from the group consisting of surgery, chemotherapy, radiation therapy, hormonal therapy, targeted therapy and palliative care, or a combination thereof.

In a particular aspect, the patients are selected at step c) for a cancer treatment, at the exclusion of an immunotherapy, when two, preferably three, more preferably four, even more preferably five of the percentages selected from the group constituted of DPP4⁺ CAFs percentage, OX40L⁺ CAFs percentage, CD73⁺ CAFs percentage, PDL2⁺ CAFs percentage, JAM2⁺ CAFs percentage, and B7H3⁺ CAFs percentage, are high.

### Comparison to a reference expression level

Preferably, the method further comprises a step of comparing the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in the cancer sample of the patient to reference level(s). Level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs under their reference level(s) are predictive of the responsiveness of said patient to an immunotherapy treatment. On the opposite, level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs above their reference level(s) are predictive of the inefficacy or lower efficacy of an immunotherapy treatment on the cancer of said patient.

The method disclosed herein for selecting a patient affected with a tumor for an immunotherapy treatment or for predicting the response of a subject affected with a tumor to an immunotherapy treatment, wherein the method comprises:
(a) detecting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient;
(b) determining the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample, wherein the responsiveness of said patient to an immunotherapy treatment is inversely proportional to the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in the cancer sample;
(c) comparing the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample to reference level(s), wherein level(s) lower than their reference level(s) are predictive of the responsiveness of said patient to an immunotherapy treatment, and/or level(s) higher than their reference level(s) are predictive of the inefficacy or lower efficacy of an immunotherapy treatment on the cancer of said patient; and
(d) optionally, selecting patients with level(s) lower than their reference level(s) as suitable for an immunotherapy treatment and/or selecting patients with level(s) higher than their reference level(s) for a cancer treatment excluding immunotherapy.

Optionally, the method may further comprise a step of providing a cancer sample from said patient before the step (a).

In a particular aspect, the patients are selected at step (d) as suitable for an immunotherapy when two, preferably three, more preferably four, even more preferably five of the levels selected from the group constituted of DPP4⁺ CAFs level, OX40L⁺ CAFs level, CD73⁺ CAFs level, PDL2⁺ CAFs level, JAM2⁺ CAFs level, and B7H3⁺ CAFs level, are lower than their reference level(s).

In another particular aspect, the patients are selected at step d) for a treatment, at the exclusion of an immunotherapy, when two, preferably three, more preferably four, even more preferably five of the levels selected from the group constituted of DPP4⁺ CAFs level, OX40L⁺ CAFs level, CD73⁺ CAFs level, PDL2⁺ CAFs level, JAM2⁺ CAFs level, and B7H3⁺ CAFs level, are higher than their reference level(s).

The cancer treatment may be selected from the group consisting of surgery, chemotherapy, radiation therapy, hormonal therapy, targeted therapy and palliative care, or a combination thereof.

In a particular aspect, the present disclosure concerns a method (which is not claimed) for providing data useful for selecting a patient affected with a tumor for an immunotherapy treatment or with an antitumor treatment excluding immunotherapy or for determining whether a patient affected with a tumor is susceptible to benefit from an immunotherapy treatment or with an antitumor treatment excluding immunotherapy, wherein the method comprises providing a cancer sample from said patient, determining the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said sample, comparing the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs to their reference level(s), wherein level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs lower than their reference level(s) is predictive that an immunotherapy treatment is indicated for said patient and optionally selecting patients with level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs lower than their reference level(s) for an immunotherapy treatment, and wherein level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs higher than their reference level(s) is predictive that an immunotherapy treatment is not indicated for said patient and optionally selecting patients with level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs higher than the reference level for an antitumor treatment excluding immunotherapy.

Optionally, the method may further comprise a step of providing a cancer sample from said patient.

Preferably, patients are selected for an immunotherapy treatment when two, preferably three, more preferably four, even more preferably five of the levels selected from the group constituted of DPP4⁺ CAFs level, OX40L⁺ CAFs level, CD73⁺ CAFs level, PDL2⁺ CAFs level, JAM2⁺ CAFs level, and B7H3⁺ CAFs level, are lower than their reference level(s).

Preferably, patients are selected for a treatment, at the exclusion of an immunotherapy treatment, when two, preferably three, more preferably four, even more preferably five of the levels selected from the group constituted of DPP4⁺ CAFs level, OX40L⁺ CAFs level, CD73⁺ CAFs level, PDL2⁺ CAFs level, JAM2⁺ CAFs level, and B7H3⁺ CAFs level, are higher than their reference level(s).

The cancer treatment may be selected from the group consisting of surgery, chemotherapy, radiation therapy, hormonal therapy, targeted therapy and palliative care, or a combination thereof.

The present disclosure also concems a method (which is not claimed) for selecting a patient affected with a tumor for an immunotherapy treatment or for determining whether a patient affected with a tumor is susceptible to benefit from an immunotherapy treatment, wherein the method comprises determining the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient, comparing the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs to their reference level(s) and optionally selecting patients with level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs lower than their reference level(s) for an immunotherapy treatment.

Optionally, the method further comprises a previous step of providing a cancer sample from said patient.

Preferably, patients are selected for an immunotherapy treatment when two, preferably three, more preferably four, even more preferably five of the levels selected from the group constituted of DPP4⁺ CAFs level, OX40L⁺ CAFs level, CD73⁺ CAFs level, PDL2⁺ CAFs level, JAM2⁺ CAFs level, and B7H3⁺ CAFs level, are lower than their reference level(s).

The disclosure also concems a method (which is not claimed) for selecting a patient affected with a tumor for an antitumor treatment excluding immunotherapy or for determining whether a patient affected with a tumor is susceptible to benefit from an antitumor treatment excluding immunotherapy, wherein the method comprises determining the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient, comparing the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs to their reference level(s) and optionally selecting patients with level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs higher than their reference level(s) for an antitumor treatment excluding immunotherapy.

Optionally, the method further comprises a previous step of providing a cancer sample from said patient.

Preferably, patients are selected for a treatment, at the exclusion of an immunotherapy treatment, when two, preferably three, more preferably four, even more preferably five of the levels selected from the group constituted of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs, are higher than their reference level(s).

The cancer treatment may be selected from the group consisting of surgery, chemotherapy, radiation therapy, hormonal therapy, targeted therapy and palliative care.

Preferably, the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient are the percentages of these cells on the total number of cells in the cancer sample or the percentage(s) of these cells on the total number of CAFs in the cancer sample. In a most preferred aspect, the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient are the percentages of these cells on the total number of cells in the cancer sample.

Therefore, the disclosure concerns a method (which is not claimed) for selecting a patient affected with a tumor for an immunotherapy treatment or for predicting the response of a subject affected with a tumor to an immunotherapy treatment, wherein the method comprises:
(a) detecting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient;
(b) determining the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample, wherein the responsiveness of said patient to an immunotherapy treatment is inversely proportional to the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in the cancer sample;
(c) comparing the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample to reference percentage(s), wherein percentage(s) lower than their reference percentage(s) are predictive of the responsiveness of said patient to an immunotherapy treatment, and/or percentage(s) higher than their reference percentage(s) are predictive of the inefficacy or lower efficacy of an immunotherapy treatment on the cancer of said patient; and
(d) optionally, selecting patients with percentage(s) lower than their reference percentage(s) as suitable for an immunotherapy treatment and/or selecting patients with percentage(s) higher than their reference percentage(s) for a cancer treatment excluding immunotherapy.

Optionally, the method may further comprise a step of providing a cancer sample from said patient before the step (a).

Preferably, the comparison of the percentages of step (c) is predictive of the responsiveness of said patient to an immunotherapy treatment and/or patients are selected at step (d) as suitable for an immunotherapy treatment when two, preferably three, more preferably four, even more preferably the five percentages selected from the group constituted of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs percentage, are lower than their reference percentages.

Preferably, the comparison of the percentages of step (c) is predictive of the inefficacy or lower efficacy of an immunotherapy treatment and/or patients are selected at step (d) for a cancer treatment, at the exclusion of an immunotherapy, when two, preferably three, more preferably four, even more preferably the five percentages selected from the group constituted of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs percentage, are higher than their reference percentages.

The cancer treatment may be selected from the group consisting of surgery, chemotherapy, radiation therapy, hormonal therapy, targeted therapy and palliative care, or a combination thereof.

Methods for the determination of reference levels, in particular reference percentages, are well known from the man skilled in the art. Preferably, the reference percentages are determined by measuring the percentage of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in the cancer samples from cohorts of patients from who the responsiveness to immunotherapy treatment is known. These percentages can be determined as disclosed in the experimental part or by analysis of cohorts of patients. Preferably, the analysis of cohorts of patients relies on an immunochemistry of cancer samples of the patients, preferably a part of the patients of said cohort are responsive to immunotherapy and a part of the patients of said cohort are irresponsive to immunotherapy. Alternatively, the reference percentages are determined by measuring the percentage of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in patient that do not suffer from cancer.

The reference percentage for DPP4⁺ CAFs in a cancer sample is comprised between 1% and 70%, preferably between 5% and 40%, more preferably between 10% and 30%, still more preferably between 15% and 25%, yet more preferably the reference percentage for DPP4⁺ CAFs in a cancer sample is of about 20%, even more preferably the reference percentage for DPP4⁺ CAFs in a cancer sample is of 20%. Alternatively, the reference percentage for DPP4⁺ CAFs in a cancer sample is comprised between 1% and 5%, 5% and 10%, 10% and 15%, 15% and 20%, 20% and 25%, 25% and 30%, 30% and 35%, 35% and 40%, 40% and 45%, 45% and 50%, 50% and 60%, and/or 60% and 70%.

In the context of the present invention, the percentage of DPP4⁺ CAFs is considered to be lower than its reference percentage if it is at least, when applicable, 0.1, 0.2, 0.3, 0.4, 0.5, 1, 2, 3, 4, 5, 6, 7, 8, 9, 10 % under the reference percentage.

In the context of the present invention, the percentage of DPP4⁺ CAFs is considered to be higher than its reference percentage if it is at least 0.1, 0.2, 0.3, 0.4, 0.5, 1, 2, 3, 4, 5, 6, 7, 8, 9, or 10% over the reference percentage.

The reference percentage for OX40L⁺ CAFs in a cancer sample is comprised between 1% and 70%, preferably between 5% and 40%, more preferably between 10% and 30%, still more preferably between 15% and 25%, yet more preferably the reference percentage for OX40L⁺ CAFs in a cancer sample is of about 20%, even more preferably the reference percentage for OX40L⁺ CAFs in a cancer sample is of 18%. Alternatively, the reference percentage for OX40L⁺ CAFs in a cancer sample is comprised between 1% and 5%, 5% and 10%, 10% and 15%, 15% and 20%, 20% and 25%, 25% and 30%, 30% and 35%, 35% and 40%, 40% and 45%, 45% and 50%, 50% and 60%, and/or 60% and 70%.

In the context of the present disclosure, the percentage of OX40L⁺ CAFs is considered to be lower than its reference percentage if it is at least 0.1, 0.2, 0.3, 0.4, 0.5, 1, 2, 3, 4, 5, 6, 7, 8, 9, 10 %, when applicable, under the reference percentage.

In the context of the present disclosure, the percentage of OX40L⁺ CAFs is considered to be higher than its reference percentage if it is at least 0.1, 0.2, 0.3, 0.4, 0.5, 1, 2, 3, 4, 5, 6, 7, 8, 9, or 10% over the reference percentage.

The reference percentage for CD73⁺ CAFs in a cancer sample is comprised between 1% and 75%, preferably between 5% and 65%, more preferably between 10% and 60%, still more preferably between 20% and 55%, yet more preferably between 40% and 50%, even more preferably the reference percentage for CD73⁺ CAFs in a cancer sample is of about 45%, and in a most preferred aspect the reference percentage for CD73⁺ CAFs in a cancer sample is of 48%. Alternatively, the reference percentage for CD73⁺ CAFs in a cancer sample is comprised between 1% and 5%, 5% and 10%, 10% and 15%, 15% and 20%, 20% and 25%, 25% and 30%, 30% and 35%, 35% and 40%, 40% and 45%, 45% and 50%, 50% and 60%, and/or 60% and 75%.

In the context of the present disclosure, the percentage of CD73⁺ CAFs is considered to be lower than its reference percentage if it is at least 0.1, 0.2, 0.3, 0.4, 0.5, 1, 2, 3, 4, 5, 6, 7, 8, 9, or 10%, when applicable, under the reference percentage.

In the context of the present disclosure, the percentage of CD73⁺ CAFs is considered to be higher than its reference percentage if it is at least 0.1, 0.2, 0.3, 0.4, 0.5, 1, 2, 3, 4, 5, 6, 7, 8, 9, or 10% over the reference percentage.

The reference percentage for PDL2⁺ CAFs in a cancer sample is comprised between 1% and 70%, preferably between 5% and 50%, more preferably between 5% and 40%, still more preferably between 5% and 30%, yet more preferably between 5% and 20%, even more preferably the reference percentage for PDL2⁺ CAFs in a cancer sample is of about 10%, and in a most preferred aspect the reference percentage for PDL2⁺ CAFs in a cancer sample is of 9%. Alternatively, the reference percentage for PDL2⁺ CAFs in a cancer sample is comprised between 1% and 5%, 5% and 10%, 10% and 15%, 15% and 20%, 20% and 25%, 25% and 30%, 30% and 35%, 35% and 40%, 40% and 45%, 45% and 50%, 50% and 60%, and/or 60% and 70%.

In the context of the present disclosure, the percentage of PDL2⁺ CAFs is considered to be lower than its reference percentage if it is at least 0.1, 0. 2, 0.3, 0.4, 0.5, 1, 2, 3, 4, 5, 6, 7, 8, 9, or 10% under, when applicable, the reference percentage.

In the context of the present disclosure, the percentage of PDL2⁺ CAFs is considered to be higher than its reference percentage if it is at least 0.1, 0. 2, 0.3, 0.4, 0.5, 1, 2, 3, 4, 5, 6, 7, 8, 9, or 10% over the reference percentage.

The reference percentage for B7H3⁺ CAFs in a cancer sample is comprised between 1% and 70%, preferably between 5% and 50%, more preferably between 10% and 45%, still more preferably between 20 % and 40%, yet more preferably the reference percentage for B7H3⁺ CAFs in a cancer sample is of about 30%, even more preferably the reference percentage for B7H3⁺ CAFs in a cancer sample is of 32%. Alternatively, the reference percentage for B7H3⁺ CAFs in a cancer sample is comprised between 1% and 5%, 5% and 10%, 10% and 15%, 15% and 20%, 20% and 25%, 25% and 30%, 30% and 35%, 35% and 40%, 40% and 45%, 45% and 50%, 50% and 60%, and/or 60% and 70%.In the context of the present disclosure, the percentage of B7H3⁺ CAFs is considered to be lower than its reference percentage if it is at least 0.1, 0.2, 0.3, 0.4, 0.5, 1, 2, 3, 4, 5, 6, 7, 8, 9, or 10% under, when applicable, the reference percentage.

In the context of the present disclosure, the percentage of B7H3⁺ CAFs is considered to be higher than its reference percentage if it is at least 0.1, 0.2, 0.3, 0.4, 0.5, 1, 2, 3, 4, 5, 6, 7, 8, 9, or 10% over the reference percentage.

The reference percentage for JAM2⁺ CAFs in a cancer sample is comprised between 1% and 70%, preferably between 5% and 50%, more preferably between 10% and 45%, still more preferably between 20 % and 40%, yet more preferably the reference percentage for JAM2⁺ CAFs in a cancer sample is of about 30%, even more preferably the reference percentage for JAM2⁺ CAFs in a cancer sample is of 35%. Altematively, the reference percentage for JAM2⁺ CAFs in a cancer sample is comprised between 1% and 5%, 5% and 10%, 10% and 15%, 15% and 20%, 20% and 25%, 25% and 30%, 30% and 35%, 35% and 40%, 40% and 45%, 45% and 50%, 50% and 60%, and/or 60% and 70%.In the context of the present disclosure, the percentage of JAM2⁺ CAFs is considered to be lower than its reference percentage if it is at least 0.1, 0.2, 0.3, 0.4, 0.5, 1, 2, 3, 4, 5, 6, 7, 8, 9, or 10% under, when applicable, the reference percentage.

In the context of the present disclosure, the percentage of JAM2⁺ CAFs is considered to be higher than its reference percentage if it is at least 0.1, 0.2, 0.3, 0.4, 0.5, 1, 2, 3, 4, 5, 6, 7, 8, 9, or 10% over the reference percentage.

In an aspect, the method for selecting a patient according to the disclosure comprises:
(a) detecting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient;
(b) determining the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample, wherein a percentage of DPP4⁺ CAFs lower than 40%, preferably lower than 30%, more preferably lower than 20%, still more preferably lower than 10%, yet more preferably lower than 5%, even more preferably lower than 1%, and/or a percentage of OX40L⁺ CAFs lower than 40%, preferably lower than 30%, more preferably lower than 20%, still more preferably lower than 10%, yet more preferably lower than 5%, even more preferably lower than 1%, and/or a percentage of CD73⁺ CAFs lower than 60%, preferably lower than 50%, more preferably lower than 30%, still more preferably lower than 20%, yet more preferably lower than 10%, even more preferably lower than 5%, and most preferably lower than 1%, and/or a percentage of PDL2⁺ CAFs lower than 40%, preferably lower than 30%, more preferably lower than 20%, still more preferably lower than 10%, yet more preferably lower than 5%, even more preferably lower than 1%, and/or a percentage of JAM2⁺ CAFs lower than 50%, preferably lower than 40%, more preferably lower than 30%, still more preferably lower than 20%, yet more preferably lower than 10%, even more preferably lower than 5%, and most preferably lower than 1%, and/or a percentage of B7H3⁺ CAFs lower than 50%, preferably lower than 40%, more preferably lower than 30%, still more preferably lower than 20%, yet more preferably lower than 10%, even more preferably lower than 5%, and most preferably lower than 1%, is predictive of the responsiveness of said patient to an immunotherapy treatment, and wherein a percentage of DPP4⁺ CAFs higher than 1%, preferably higher than 5%, more preferably higher than 10%, still more preferably higher than 20%, yet more preferably higher than 30%, even more preferably higher than 40%, and/or a percentage of OX40L⁺ higher than 1%, preferably higher than 5%, more preferably higher than 10%, still more preferably higher than 20%, yet more preferably higher than 30%, even more preferably higher than 40%, and/or a percentage of CD73⁺ CAFs higher than 1%, preferably higher than 5%, more preferably higher than 10%, still more preferably higher than 20%, yet more preferably higher than 30%, even more preferably higher than 50%, and most preferably higher than 60% and/or a percentage of PDL2⁺ CAFs higher than 1%, preferably higher than 5%, more preferably higher than 10%, still more preferably higher than 20%, yet more preferably higher than 30%, even more preferably higher than 40%, and/or a percentage of JAM2⁺ CAFs higher than 1%, preferably higher than 5%, more preferably higher than 10%, still more preferably higher than 20%, yet more preferably higher than 30%, even more preferably higher than 40%, and most preferably higher than 50% and/or a percentage of B7H3⁺ CAFs higher than 1%, preferably higher than 5%, more preferably higher than 10%, still more preferably higher than 20%, yet more preferably higher than 30%, even more preferably higher than 40%, and most preferably higher than 50% is predictive of the inefficacy or lower efficacy of an immunotherapy treatment on the cancer of said patient; and
(c) optionally, selecting patients with a percentage of DPP4⁺ CAFs lower than 40%, preferably lower than 30%, more preferably lower than 20%, still more preferably lower than 10%, yet more preferably lower than 5%, even more preferably lower than 1%, and/or a percentage of OX40L⁺ CAFs lower than 40%, preferably lower than 30%, more preferably lower than 20%, still more preferably lower than 10%, yet more preferably lower than 5%, even more preferably lower than 1%, and/or a percentage of CD73⁺ CAFs lower than 60%, preferably lower than 50%, more preferably lower than 30%, still more preferably lower than 20%, yet more preferably lower than 10%, even more preferably lower than 5%, and most preferably lower than 1% and/or a percentage of PDL2⁺ CAFs lower than 40%, preferably lower than 30%, more preferably lower than 20%, still more preferably lower than 10%, yet more preferably lower than 5%, even more preferably lower than 1%, and/or a percentage of JAM2⁺ CAFs lower than 50%, preferably lower than 40%, more preferably lower than 30%, still more preferably lower than 20%, yet more preferably lower than 10%, even more preferably lower than 5%, and most preferably lower than 1%, and/or a percentage of B7H3⁺ CAFs lower than 50%, preferably lower than 40%, more preferably lower than 30%, still more preferably lower than 20%, yet more preferably lower than 10%, even more preferably lower than 5%, and most preferably lower than 1%, as suitable for an immunotherapy treatment, and/or selecting patients with a percentage of DPP4⁺ CAFs higher than 1%, preferably higher than 5%, more preferably higher than 10%, still more preferably higher than 20%, yet more preferably higher than 30%, even more preferably higher than 40%, and/or a percentage of OX40L⁺ higher than 1%, preferably higher than 5%, more preferably higher than 10%, still more preferably higher than 20%, yet more preferably higher than 30%, even more preferably higher than 40%, and/or a percentage of CD73⁺ CAFs higher than 1%, preferably higher than 5%, more preferably higher than 10%, still more preferably higher than 20%, yet more preferably higher than 30%, even more preferably higher than 50%, and most preferably higher than 60% and/or a percentage of PDL2⁺ CAFs higher than 1%, preferably higher than 5%, more preferably higher than 10%, still more preferably higher than 20%, yet more preferably higher than 30%, even more preferably higher than 40%, and/or a percentage of JAM2⁺ CAFs higher than 1%, preferably higher than 5%, more preferably higher than 10%, still more preferably higher than 20%, yet more preferably higher than 30%, even more preferably higher than 40%, and most preferably higher than 50% and/or a percentage of B7H3⁺ CAFs higher than 1%, preferably higher than 5%, more preferably higher than 10%, still more preferably higher than 20%, yet more preferably higher than 30%, even more preferably higher than 40%, and most preferably higher than 50% for a cancer treatment excluding immunotherapy.

In an aspect, the method for selecting a patient according to the disclosure comprises:
(a) detecting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient;
(b) determining the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample, wherein a percentage of DPP4⁺ CAFs lower than 20%, and/or a percentage of OX40L⁺ CAFs lower than 18%, and/or a percentage of CD73⁺ CAFs lower than 48%, and/or a percentage of PDL2⁺ CAFs lower than 9%, and/or a percentage of JAM2⁺ CAFs lower than 35%, and/or a percentage of B7H3⁺ CAFs lower than 32%, is predictive of the responsiveness of said patient to an immunotherapy treatment, and wherein a percentage of DPP4⁺ CAFs higher than 20%, and/or a percentage of OX40L⁺higher than 18%, and/or a percentage of CD73⁺ CAFs higher than 48%, and/or a percentage of PDL2⁺ CAFs higher than 9%, and/or a percentage of JAM2⁺ CAFs higher than 35%, and/or a percentage of B7H3⁺ CAFs higher than 32%, is predictive of the inefficacy or lower efficacy of an immunotherapy treatment on the cancer of said patient; and
(c) optionally, selecting patients with wherein a percentage of DPP4⁺ CAFs lower than 20%, and/or a percentage of OX40L⁺ CAFs lower than 18%, and/or a percentage of CD73⁺ CAFs lower than 48%, and/or a percentage of PDL2⁺ CAFs lower than 9%, and/or a percentage of B7H3⁺ CAFs lower than 32%, and/or a percentage of JAM2⁺ CAFs lower than 35%, as suitable for an immunotherapy treatment, and/or selecting patients with a percentage of OX40L⁺ higher than 18%, and/or a percentage of CD73⁺ CAFs higher than 48%, and/or a percentage of PDL2⁺ CAFs higher than 9%, and/or a percentage of JAM2⁺ CAFs higher than 35%, and/or a percentage of B7H3⁺ CAFs higher than 32%, for a cancer treatment excluding immunotherapy.

In one embodiment, the method for selecting a patient according to the invention comprises:
(a) detecting DPP4⁺ CAFs in a cancer sample from said patient;
(b) determining the percentage of DPP4⁺ CAFs in said cancer sample wherein a percentage lower than 30%, preferably lower than 20%, more preferably lower than 10%, still more preferably lower than 5%, even more preferably lower than 1%, is predictive of the responsiveness of said patient to an immunotherapy treatment, and/or wherein a percentage higher than 1%, preferably higher than 5%, more preferably higher than 10%, still more preferably higher than 20%, even more preferably higher than 30%, is predictive of the inefficacy or lower efficacy of an immunotherapy treatment on the cancer of said patient; and
(c) optionally, selecting patients with a percentage of DPP4⁺ CAFs lower than 30%, preferably lower than 20%, more preferably lower than 10%, still more preferably lower than 5%, even more preferably lower than 1%, as suitable for an immunotherapy treatment and/or selecting patients with a percentage of DPP4⁺ CAFs higher than 1%, preferably higher than 5%, more preferably higher than 10%, still more preferably higher than 20%, even more preferably higher than 30% for a cancer treatment excluding immunotherapy.

In an altemative embodiment, the method for selecting a patient according to the invention comprises:
(a) detecting DPP4⁺ CAFs in a cancer sample from said patient;
(b) determining the percentage of DPP4⁺ CAFs in said cancer sample wherein a percentage lower than 20% is predictive of the responsiveness of said patient to an immunotherapy treatment, and/or wherein a percentage higher than 20% is predictive of the inefficacy or lower efficacy of an immunotherapy treatment on the cancer of said patient; and
(c) optionally, selecting patients with a percentage of DPP4⁺ CAFs lower than 20% as suitable for an immunotherapy treatment and/or selecting patients with a percentage of DPP4⁺ CAFs higher than 20% for a cancer treatment excluding immunotherapy.

In yet another embodiment, the method for selecting a patient according to the invention comprises:
(a) detecting DPP4⁺ CAFs, OX40L⁺ CAFs, CD73⁺ CAFs, PDL2⁺ CAFs, JAM2⁺ CAFs, and B7H3⁺ CAFs in a cancer sample from said patient;
(b) determining the percentage(s) of DPP4⁺ CAFs, OX40L⁺ CAFs, CD73⁺ CAFs, PDL2⁺ CAFs, JAM2⁺ CAFs, and B7H3⁺ CAFs in said cancer sample, wherein a combination of two, preferably of three, more preferably of four, even more preferably of five, of the followings:
   - a percentage of DPP4⁺ CAFs lower than 40%, preferably lower than 30%, more preferably lower than 20%, still more preferably lower than 10%, yet more preferably lower than 5%, even more preferably lower than 1%;
   - a percentage of OX40L⁺ CAFs lower than 40%, preferably lower than 30%, more preferably lower than 20%, still more preferably lower than 10%, yet more preferably lower than 5%, even more preferably lower than 1%; and
   - a percentage of CD73⁺ CAFs lower than 60%, preferably lower than 50%, more preferably lower than 30%, still more preferably lower than 20%, yet more preferably lower than 10%, even more preferably lower than 5%, most preferably lower than 1%;
   - a percentage of PDL2⁺ CAFs lower than 40%, preferably lower than 30%, more preferably lower than 20%, still more preferably lower than 10%, yet more preferably lower than 5%, even more preferably lower than 1%;
   - a percentage of JAM2⁺ CAFs lower than 50%, preferably lower than 40%, more preferably lower than 30%, still more preferably lower than 20%, yet more preferably lower than 10%, even more preferably lower than 5%, most preferably lower than 1%;
   - a percentage of B7H3⁺ CAFs lower than 50%, preferably lower than 40%, more preferably lower than 30%, still more preferably lower than 20%, yet more preferably lower than 10%, even more preferably lower than 5%, most preferably lower than 1%;
   is predictive of the responsiveness of said patient to an immunotherapy treatment, and wherein a combination of two, preferably of three, more preferably of four, even more preferably of five, of the followings:
   - a percentage of DPP4⁺ CAFs higher than 1%, preferably higher than 5%, more preferably higher than 10%, still more preferably higher than 20%, yet more preferably higher than 30%, even more preferably higher than 40%;
   - a percentage of OX40L⁺ higher than 1%, preferably higher than 5%, more preferably higher than 10%, still more preferably higher than 20%, yet more preferably higher than 30%, even more preferably higher than 40%; and
   - a percentage of CD73⁺ CAFs higher than 1%, preferably higher than 5%, more preferably higher than 10%, still more preferably higher than 20%, yet more preferably higher than 30%, even more preferably higher than 50%, most preferably higher than 60%;
   - a percentage of PDL2⁺ CAFs higher than 1%, preferably higher than 5%, more preferably higher than 10%, still more preferably higher than 20%, yet more preferably higher than 30%, even more preferably higher than 40%;
   - a percentage of JAM2⁺ CAFs higher than 1%, preferably higher than 5%, more preferably higher than 10%, still more preferably higher than 20%, yet more preferably higher than 30%, even more preferably higher than 40%, most preferably higher than 50%;
   - a percentage of B7H3⁺ CAFs higher than 1%, preferably higher than 5%, more preferably higher than 10%, still more preferably higher than 20%, yet more preferably higher than 30%, even more preferably higher than 40%, most preferably higher than 50%;
   is predictive of the inefficacy or lower efficacy of an immunotherapy treatment on the cancer of said patient; and
(c) optionally, selecting patients with a combination of two, preferably of three, more preferably of four, even more preferably of five, of the followings:
   - a percentage of DPP4⁺ CAFs lower than 40%, preferably lower than 30%, more preferably lower than 20%, still more preferably lower than 10%, yet more preferably lower than 5%, even more preferably lower than 1%;
   - a percentage of OX40L⁺ CAFs lower than 40%, preferably lower than 30%, more preferably lower than 20%, still more preferably lower than 10%, yet more preferably lower than 5%, even more preferably lower than 1%; and
   - a percentage of CD73⁺ CAFs lower than 60%, preferably lower than 50%, more preferably lower than 30%, still more preferably lower than 20%, yet more preferably lower than 10%, even more preferably lower than 5%, most preferably lower than 1%;
   - a percentage of PDL2⁺ CAFs lower than 40%, preferably lower than 30%, more preferably lower than 20%, still more preferably lower than 10%, yet more preferably lower than 5%, even more preferably lower than 1%;
   - a percentage of JAM2⁺ CAFs lower than 50%, preferably lower than 40%, more preferably lower than 30%, still more preferably lower than 20%, yet more preferably lower than 10%, even more preferably lower than 5%, most preferably lower than 1%;
   - a percentage of B7H3⁺ CAFs lower than 50%, preferably lower than 40%, more preferably lower than 30%, still more preferably lower than 20%, yet more preferably lower than 10%, even more preferably lower than 5%, most preferably lower than 1%;
   as suitable for an immunotherapy treatment, and/or selecting patients with a combination of two, preferably of three, more preferably of four, even more preferably of five, of the followings:
   - a percentage of DPP4⁺ CAFs higher than 1%, preferably higher than 5%, more preferably higher than 10%, still more preferably higher than 20%, yet more preferably higher than 30%, even more preferably higher than 40%;
   - a percentage of OX40L⁺ higher than 1%, preferably higher than 5%, more preferably higher than 10%, still more preferably higher than 20%, yet more preferably higher than 30%, even more preferably higher than 40%; and
   - a percentage of CD73⁺ CAFs higher than 1%, preferably higher than 5%, more preferably higher than 10%, still more preferably higher than 20%, yet more preferably higher than 30%, even more preferably higher than 50%, most preferably higher than 60%;
   - a percentage of PDL2⁺ CAFs higher than 1%, preferably higher than 5%, more preferably higher than 10%, still more preferably higher than 20%, yet more preferably higher than 30%, even more preferably higher than 40%;
   - a percentage of JAM2⁺ CAFs higher than 1%, preferably higher than 5%, more preferably higher than 10%, still more preferably higher than 20%, yet more preferably higher than 30%, even more preferably higher than 40%, most preferably higher than 50%;
   - a percentage of B7H3⁺ CAFs higher than 1%, preferably higher than 5%, more preferably higher than 10%, still more preferably higher than 20%, yet more preferably higher than 30%, even more preferably higher than 40%, most preferably higher than 50%;
   for a cancer treatment excluding immunotherapy.

In yet another embodiment, the method for selecting a patient comprises:
(a) detecting DPP4⁺, CD73⁺, B7H3⁺, OX40L⁺, JAM2⁺ and PDL2⁺ CAFs in a cancer sample from said patient;
(b) determining the percentage(s) of DPP4⁺ CAFs, CD73⁺ CAFs, B7H3⁺ CAFs, OX40L⁺ CAFs, JAM2⁺ CAFs and PDL2⁺ CAFs in said cancer sample, wherein the combination of a percentage of DPP4⁺ CAFs lower than 30%, preferably lower than 20%, more preferably lower than 10%, still more preferably lower than 5%, even more preferably lower than 1%, and a percentage of OX40L⁺ CAFs lower than 30%, preferably lower than 18%, more preferably lower than 10%, still more preferably lower than 5%, even more preferably lower than 1%, and a percentage of CD73⁺ CAFs lower than 60%, preferably lower than 48%, more preferably lower than 30%, still more preferably lower than 10%, yet more preferably lower than 5%, even more preferably lower than 1%, and a percentage of PDL2⁺ CAFs lower than 30%, preferably lower than 20%, more preferably lower than 9%, still more preferably lower than 5%, even more preferably lower than 1%, and a percentage of JAM2⁺ CAFs lower than 50%, preferably lower than 35%, more preferably lower than 20%, still more preferably lower than 10%, yet more preferably lower than 5%, even more preferably lower than 1% and a percentage of B7H3⁺ CAFs lower than 50%, preferably lower than 40%, more preferably lower than 32%, still more preferably lower than 10%, yet more preferably lower than 5%, even more preferably lower than 1%, is predictive of the responsiveness of said patient to an immunotherapy treatment, and wherein the combination of a percentage of DPP4⁺ CAFs higher than 1%, preferably higher than 5%, more preferably higher than 10%, still more preferably higher than 20%, even more preferably higher than 30%, and a percentage of OX40L⁺ higher than 1%, preferably higher than 5%, more preferably higher than 10%, still more preferably higher than 18%, even more preferably higher than 30%, and a percentage of CD73⁺ CAFs higher than 1%, preferably higher than 5%, more preferably higher than 10%, still more preferably higher than 30%, yet more preferably higher than 48%, even more preferably higher than 60%, and a percentage of PDL2⁺ CAFs higher than 1%, preferably higher than 5%, more preferably higher than 9%, still more preferably higher than 20%, even more preferably higher than 30%, and a percentage of JAM2⁺ CAFs higher than 1%, preferably higher than 5%, more preferably higher than 10%, still more preferably higher than 35%, yet more preferably higher than 40% and even more preferably higher than 50%, and a percentage of B7H3⁺ CAFs higher than 1%, preferably higher than 5%, more preferably higher than 10%, still more preferably higher than 32%, yet more preferably higher than 40% and even more preferably higher than 50%, is predictive of the inefficacy or lower efficacy of an immunotherapy treatment on the cancer of said patient; and
(c) optionally, selecting patients with the combination of a percentage of DPP4⁺ CAFs lower than 30%, preferably lower than 20%, more preferably lower than 10%, still more preferably lower than 5%, even more preferably lower than 1%, and a percentage of OX40L⁺ CAFs lower than 30%, preferably lower than 18%, more preferably lower than 10%, still more preferably lower than 5%, even more preferably lower than 1%, and a percentage of CD73⁺ CAFs lower than 60%, preferably lower than 48%, more preferably lower than 30%, still more preferably lower than 10%, yet more preferably lower than 5%, even more preferably lower than 1%, and a percentage of PDL2⁺ CAFs lower than 30%, preferably lower than 20%, more preferably lower than 9%, still more preferably lower than 5%, even more preferably lower than 1%, and a percentage of JAM2⁺ CAFs lower than 50%, preferably lower than 35%, more preferably lower than 20%, still more preferably lower than 10%, yet more preferably lower than 5%, even more preferably lower than 1% and a percentage of B7H3⁺ CAFs lower than 50%, preferably lower than 40%, more preferably lower than 32%, still more preferably lower than 10%, yet more preferably lower than 5%, even more preferably lower than 1%, as suitable for an immunotherapy treatment, and/or selecting patients with the combination of a percentage of DPP4⁺ CAFs higher than 1%, preferably higher than 5%, more preferably higher than 10%, still more preferably higher than 20%, even more preferably higher than 30%, and a percentage of OX40L⁺ higher than 1%, preferably higher than 5%, more preferably higher than 10%, still more preferably higher than 18%, even more preferably higher than 30%, and a percentage of CD73⁺ CAFs higher than 1%, preferably higher than 5%, more preferably higher than 10%, still more preferably higher than 30%, yet more preferably higher than 48%, even more preferably higher than 60%, and a percentage of PDL2⁺ CAFs higher than 1%, preferably higher than 5%, more preferably higher than 9%, still more preferably higher than 20%, even more preferably higher than 30%, and a percentage of JAM2⁺ CAFs higher than 1%, preferably higher than 5%, more preferably higher than 10%, still more preferably higher than 35%, yet more preferably higher than 40% and even more preferably higher than 50% and a percentage of B7H3⁺ CAFs higher than 1%, preferably higher than 5%, more preferably higher than 10%, still more preferably higher than 32%, yet more preferably higher than 40% and even more preferably higher than 50%, for a cancer treatment excluding immunotherapy.

In an alternate embodiment, the method for selecting a patient according to the invention comprises:
(a) detecting DPP4⁺ CAFs, OX40L⁺ CAFs, CD73⁺ CAFs, PDL2⁺ CAFs, JAM2⁺ CAFs, and B7H3⁺ CAFs in a cancer sample from said patient;
(b) determining the percentage(s) of DPP4⁺ CAFs, OX40L⁺ CAFs, CD73⁺ CAFs, PDL2⁺ CAFs, JAM2⁺ CAFs, and B7H3⁺ CAFs in said cancer sample, wherein the combination of a percentage of DPP4⁺ CAFs lower than 20%, a percentage of OX40L⁺ CAFs lower than 18%, a percentage of CD73⁺ CAFs lower than 48%, a percentage of PDL2⁺ CAFs lower than 9%, a percentage of JAM2⁺ CAFs lower than 35%, and a percentage of B7H3⁺ CAFs lower than 32% is predictive of the responsiveness of said patient to an immunotherapy treatment, and wherein the combination of a percentage of DPP4⁺ CAFs higher than 20%, a percentage of OX40L⁺ higher than 18%, a percentage of CD73⁺ CAFs higher than 48%, a percentage of PDL2⁺ CAFs higher than 9%, a percentage of JAM2⁺ CAFs higher than 35%, and a percentage of B7H3⁺ CAFs higher than 32% is predictive of the inefficacy or lower efficacy of an immunotherapy treatment on the cancer of said patient; and
(c) optionally, selecting patients with the combination of a percentage of DPP4⁺ CAFs lower than 20%, a percentage of OX40L⁺ CAFs lower than 18%, a percentage of CD73⁺ CAFs lower than 48%, a percentage of PDL2⁺ CAFs lower than 9%, a percentage of JAM2⁺ CAFs lower than 35%, and a percentage of B7H3⁺ CAFs lower than 32%, as suitable for an immunotherapy treatment, and/or selecting patients with the combination of a percentage of DPP4⁺ CAFs higher than 20%, a percentage of OX40L⁺higher than 18%, a percentage of CD73⁺ CAFs higher than 48%, a percentage of PDL2⁺ CAFs higher than 9%, a percentage of JAM2⁺ CAFs higher than 35%, and a percentage of B7H3⁺ CAFs higher than 32%, for a cancer treatment excluding immunotherapy.

### Cancer

The method of the invention is aimed to select a patient affected with a tumor for a treatment.

Preferably, the tumor is from a cancer selected from the group consisting of prostate cancers, lung cancers, breast cancers, gastric cancers, kidney cancers, ovarian cancers, hepatocellular cancers, osteosarcomas, melanomas, hypopharynx cancers, esophageal cancers, endometrial cancers, cervical cancers, pancreatic cancers, liver cancers, colon or colorectal cancers, neuroendocrine cancers, muscle cancers, adrenal cancers, thyroid cancers, uterine cancers, skin cancers, bladder cancers, head and neck cancers, and pediatric cancers.

More preferably, the cancer is selected from the group consisting of ovarian cancer, breast cancer, lung cancers, colorectal cancers, pancreatic cancers and pediatric cancers.

Even more preferably, the cancer is an ovarian cancer, preferably a mesenchymal ovarian cancer, in particular a high grade ovarian cancer of the serous type, or a breast cancer, preferably an invasive breast cancer and/or its metastasis, in particular axillary metastasis.

### Immunotherapy treatment

The method of the disclosure predict the efficiency of an immunotherapy treatment according to the level of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs, in the cancer sample of a patient and thus allows to select patients for an immunotherapy treatment. Indeed, a level of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in the cancer sample lower than their reference level is predictive of the efficacy of an immunotherapy treatment.

Preferably, the immunotherapy treatment is selected from the group consisting of therapeutic treatments that stimulate the patient's immune system to attack the malignant tumor cells, immunization of the patient with tumoral antigens, , adoptive T-cell therapy, immune checkpoint inhibitor treatment, and any combination thereof, preferably an immune checkpoint inhibitor treatment.

In a most preferred embodiment, the immunotherapy treatment is an immune checkpoint inhibitor treatment, preferably selected from the group consisting of an anti-CTLA-4 (cytotoxic T lymphocyte associated protein 4) therapy such as ipilimumab, PD-1 (programmed cell death protein 1) inhibitors such as nivolumab, pembrolizumab, or BGB-A317, PDL1 (programmed cell death ligand) inhibitors such as atezolizumab, avelumab, or durvalumab, LAG-3 (Lymphocyte-activation gene 3) inhibitors such as BMS-986016, TIM-3 (T-cell immunoglobulin and mucin-domain containing-3) inhibitors, TIGIT (T cell immunoreceptor with Ig and ITIM domains) inhibitors, BLTA (B- and T-lymphocyte attenuator) inhibitors, IDO1 inhibitors such as epacadostat, or a combination thereof.

### Patient, regimen and administration

The patient is an animal, preferably a mammal, even more preferably a human. However, the patient can also be a non-human animal, in particular mammals such as dogs, cats, horses, cows, pigs, sheep, donkeys, rabbits, ferrets, gerbils, hamsters, chinchillas, rats, mice, guinea pigs and non-human primates, among others, that are in need of treatment.

The human patient disclosed herein may be a human at the prenatal stage, a new-born, a child, an infant, an adolescent or an adult, in particular an adult of at least 40 years old, preferably an adult of at least 50 years old, still more preferably an adult of at least 60 years old, even more preferably an adult of at least 70 years old.

Preferably, the patient has been diagnosed with a cancer.

In a particular aspect, the patient has already received at least one line of treatment, preferably several lines of treatment.

In another particular aspect, the patient suffers from a metastatic cancer or a cancer at an advanced stage.

The cancer treatment, in particular the immunotherapy treatment disclosed herein, can be to be administered by any conventional route of administration, such as topical, enteral, oral, parenteral, intranasal, intravenous, intramuscular, subcutaneous or intraocular route of administration and the like.

Preferably, the cancer treatment, in particular the immunotherapy treatment, start no longer than a month, preferably no longer than a week, after the determination of the level of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs.

The cancer treatment, in particular the immunotherapy treatment disclosed herein, may be to be administered as a single dose or in multiple doses.

Preferably, the treatment is to be administered regularly, preferably between every day and every month, more preferably between every day and every two weeks, even more preferably between every day and every week.

The duration of treatment with the cancer treatment, in particular the immunotherapy treatment disclosed herein, is preferably comprised between 1 day and 24 weeks, more preferably between 1 day and 10 weeks, even more preferably between 1 day and 4 weeks. In a particular aspect, the treatment last as long as the cancer persists.

The amount of cancer treatment, in particular the immunotherapy treatment disclosed herein, to be administered has to be determined by standard procedure well known by those of ordinary skills in the art. Physiological data of the patient (e.g. age, size, weight, and physical general condition) and the routes of administration have to be taken into account to determine the appropriate dosage, so as a therapeutically effective amount will be to be administered to the patient.

### Method for determining the CAFs subpopulation level

The disclosure also refers to a method (which is not claimed) for determining the level of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient, wherein the method comprises:
(a) providing a cancer sample from said patient; and
(b) detecting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient;
(c) determining the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample.

The method may further comprise a step of comparing said level(s) to their reference level(s).

Preferably, the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient are the percentage of these cells on the total number of cells in the cancer sample or the percentage of these cells on the total number of CAFs in the cancer sample. In a most preferred aspect, the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient are the percentage of these cells on the total number of cells in the cancer sample.

The disclosure also concerns a method for determining the level of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient, wherein the method comprises:
(a) providing a cancer sample from said patient; and
(b) detecting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient, preferably by immunohistochemistry or FACS as described above;
(c) determining the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample.

The method may further comprise a step of comparing said percentage(s) to their reference percentage(s). The reference percentages are as described above.

The DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs are detected as described above.

### Use of an immunotherapy treatment and treatment methods

In a particular aspect, the disclosure also concerns an immunotherapy treatment, preferably an immune checkpoint inhibitor treatment, for use in the treatment of a cancer in a patient (which is not claimed) wherein the patient presents in a cancer sample:
(a) low level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs, preferably DPP4⁺ CAFs, and/or CD73⁺ CAFs, and/or B7H3⁺ CAFs, and/or OX40L⁺ CAFs, and/or JAM2⁺ CAFs, optionally DPP4⁺ CAFs, and/or CD73⁺ CAFs, and/or B7H3⁺ CAFs, and/or OX40L⁺ CAFs;
(b) no DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs, preferably no DPP4⁺ CAFs, and/or CD73⁺ CAFs, and/or B7H3⁺ CAFs, and/or OX40L⁺ CAFs, and/or JAM2⁺ CAFs, optionally no DPP4⁺ CAFs, and/or CD73⁺ CAFs, and/or B7H3⁺ CAFs, and/or OX40L⁺ CAFs; or
(c) level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs lower than their reference level(s), preferably of DPP4⁺ CAFs, and/or CD73⁺ CAFs, and/or B7H3⁺ CAFs, and/or OX40L⁺ CAFs, and/or JAM2⁺ CAFs, optionally of DPP4⁺ CAFs, and/or CD73⁺ CAFs, and/or B7H3⁺ CAFs, and/or OX40L⁺ CAFs.

Preferably, the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient are the percentage of these cells on the total number of cells in the cancer sample or the percentage of these cells on the total number of CAFs in the cancer sample. In a most preferred aspect, the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient are the percentage of these cells on the total number of cells in the cancer sample.

Thus, the disclosure also concerns an immunotherapy treatment, preferably an immune checkpoint inhibitor treatment, for use in the treatment of a cancer in a patient wherein the patient presents in a cancer sample:
(a) low percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs, preferably of DPP4⁺ CAFs, and/or CD73⁺ CAFs, and/or B7H3⁺ CAFs, and/or OX40L⁺ CAFs, and/or JAM2⁺ CAFs, optionally of DPP4⁺ CAFs, and/or CD73⁺ CAFs, and/or B7H3⁺ CAFs, and/or OX40L⁺ CAFs;
(b) no DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs^{,} preferably no DPP4⁺ CAFs, and/or CD73⁺ CAFs, and/or B7H3⁺ CAFs, and/or OX40L⁺ CAFs, and/or JAM2⁺ CAFs, optionally no DPP4⁺ CAFs, and/or CD73⁺ CAFs, and/or B7H3⁺ CAFs, and/or OX40L⁺ CAFs; or
(c) percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs lower than their reference percentage(s), preferably of DPP4⁺ CAFs, and/or CD73⁺ CAFs, and/or B7H3⁺ CAFs, and/or OX40L⁺ CAFs, and/or JAM2⁺ CAFs, optionally of DPP4⁺ CAFs, and/or CD73⁺ CAFs, and/or B7H3⁺ CAFs, and/or OX40L⁺ CAFs.

### CAFs subpopulation targeting agents

In another aspect, the disclosure also concerns a DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent, which is not claimed.

The DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent may be selected from the group consisting of agents inhibiting the immunosuppressive activity of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs , antibodies targeting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs, optionally conjugated to a cytotoxic drug, and any combination thereof.

In a first aspect, the DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent is an agent inhibiting the immunosuppressive activity of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs such as DPP4 inhibitors and/or CD73 inhibitors.

DPP4 inhibitors may be selected from the group consisting of sitagliptin, vildagliptin, saxagliptin, linagliptin, gemigliptin, anagliptin, teneligliptin, alogliptin trelagliptin, omarigliptin, Evogliptin, dutogliptin, berberine, lupeol, and combination thereof.

CD73 inhibitors may be selected from the group consisting of alpha,beta-Methyleneadenosine Sprime-diphosphate, Adenosine 5'-(alpha,beta-methylene)diphosphate, and combination thereof. CD73 inhibitors disclosed herein can also be selected from the CD73 inhibitors disclosed in WO 2016/131950 and WO 2017/064043.

In a second aspect, the DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent is an antibody targeting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs, such as anti-DPP4 antibodies, anti-OX40L antibodies, anti-CD73 antibodies, anti-PDL2 antibodies, anti-JAM2 antibodies, anti-B7H3 antibodies, and combination thereof.

The antibody disclosed herein can be any kind of antibody. In particular, the antibody can comprise, consist or consist essentially in a classical Y-shaped antibody with two heavy chains and light chains or a fragment thereof. Preferably said fragment comprises the antigen binding or variable region of the antibody. Said fragment may be selected, without limitation, from the group consisting in Fv, Fab, Fab', F(ab)2, F(ab')2, F(ab)3, Fv, single-chain Fv (scFv), di-scFvs or sc(Fv)2, dsFv, Fd, dAb, CDRs, VH, VL, minibodies, diabodies, and multi-specific antibodies formed from antibodies fragments.

The antibody disclosed herein can also comprise, consist or consist essentially in a heavy-chain antibody. Preferably a heavy-chain antibody is selected from the heavy-chain antibodies from camelids or from cartilaginous fishes. More preferably, the antibody is a heavy-chain antibody derived from camelids. Camelids antibody encompasses in particular dromedary, camel, lama and alpaca. Preferably, the antibody is a heavy-chain antibody derived from lama.

In a preferred aspect, the antibody comprises, consists, or consists essentially in a single domain antibody or a fragment thereof. The single domain antibody can derive from a VHH or a V-NAR, preferably from a VHH. In particular, the antibody disclosed herein can be a humanized single domain antibody, in particular a humanized VHH, or a fragment thereof.

Preferably, said fragment of the single domain antibody comprises the three CDRs.

Optionally, the antibody disclosed herein is a single domain antibody fused to an Fc region, preferably an Fc region selected from the group consisting in IgA, IgD, IgE, IgG, and IgM Fc regions, more preferably an IgG Fc region.

Preferably, the Fc region is selected from human, mouse and rabbit Fc regions. Even more preferably, the antibody disclosed herein is a single domain antibody fused to a human Fc region, even more preferably a single domain antibody fused to a human IgG Fc region.

The antibody disclosed herein may be a monomeric antibody or a multimeric antibody. In particular, the antibody disclosed herein is a monomeric antibody.

The antibody disclosed herein may also be a multimeric antibody. When the antibody is a multimeric classical Y-shape antibody, it is preferably a dimer or a pentamer. Alternatively, the antibody is a multimeric single domain antibody, preferably a dimerized single domain antibody.

In a multimeric antibody, the variable domains of the different monomers can be identical (i.e. homomeric) or different (i.e. heteromeric). Preferably, the multimeric antibody disclosed herein is homomeric. When the multimeric antibody disclosed herein is heteromeric, the variable domains of the different monomers can all bind the same protein. Alternatively, they can bind different proteins.

The antibody disclosed herein can be monoclonal or polyclonal. Preferably, the antibody disclosed herein is monoclonal.

The antibody disclosed herein comprises at least a variable domain. It may comprise several variable domains, in particular when the antibody is multimeric.

The antibody disclosed herein may present the capacity to inhibit the immunosuppressive activity of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs. For instance, it can be a neutralizing antibody, it can block the binding of DPP4, OX40L, CD73, PDL2, JAM2, or B7H3, to their binding partner, e.g. substrate or receptor.

In a third aspect, the DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent is an antibody targeting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs and conjugated to a drug, preferably a cytotoxic drug.

In a preferred aspect, the DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent is an anti-DPP4 antibody conjugated to a drug, preferably a cytotoxic drug.

In another preferred aspect, the DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent is an anti-OX40L antibody conjugated to a drug, preferably a cytotoxic drug.

In yet another preferred aspect, the DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent is an anti-CD73 antibody conjugated to a drug, preferably a cytotoxic drug.

In still another preferred aspect, the DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent is an anti-PDL2 antibody conjugated to a drug, preferably a cytotoxic drug.

In another preferred aspect, the DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent is an anti-B7H3 antibody conjugated to a drug, preferably a cytotoxic drug.

The drug disclosed herein is preferably a cytotoxic drug. As used herein, the term "cytotoxic drug" refers to a molecule that when entering in contact with a cell, eventually upon internalization into the cell, alters a cell function (e.g. cell growth and/or proliferation and/or differentiation and/or metabolism such as protein and/or DNA synthesis) in a detrimental way or leads to cell death. As used herein, the term "cytotoxic drug" encompasses toxins, in particular cytotoxins.

The cytotoxic drug disclosed herein may be selected from the group consisting of dolastatins such as dolastin 10, dolastin 15, auristatin E, auristatin EB (AEB), auristatin EFP (AEFP), monomethyl auristatin F (MMAF), monomethylauristatin-D (MMAD), monomethyl auristatin E (MMAE), and 5-benzoylvaleric acid-AE ester (AEVB), maytansines such ansamitocin, mertansine (also called emtansine or DM1) and ravtansine (also called soravtansine or DM4), antracyclins such as daunorubicin, epirubicin, pirarubicin, idarubicin, zorubicin, cerubidin, aclarubicin, adriblastin, doxorubicin, mitoxantrone,daunoxome, nemorubicin and PNU-159682, calicheamicins such as calicheamicin beta 1Br, calicheamicin gamma 1Br, calicheamicin alpha 21, calicheamicin alpha 31, calicheamicin beta 1I, calicheamicin gamma 1L, calicheamicin delta 1I and ozogamicin, esperamicins such as esperamicin A1, neocarzinostatins, bleomycin, duocarymycins such as CC-1065 and duocarmycin A, pyrrolobenzodiazepines such as anthramycin, abbeymycin, chicamycin, DC-81, mazethramycin, neothramycins A and B, porothramycin prothracarcin, sibanomicin (DC-102), sibiromycin and tomamycin, pyrrolobenzodiazepine dimers (or PBD), indolino-benzodiazepines, indolino-benzodiazepine dimers, α-amanitins, abraxane, actinomycin, aldesleukin, altretamine, alitretinoin, amsacrine, anastrozole, arsenic, asparaginase, azacitidine, azathioprine, bexarotene, bendamustine, bicalutamide, bortezomib, busulfan, capecitabine, carboplatin, carmustine, chlorambucil, cisplatin, cladribine, clofarabine, cyclophosphamide, cytarabine, chloramphenicol, ciclosporin, cidofovir, coal tar containing products, colchicine, dacarbazine, dactinomycin, danazol, dasatinib, diethylstilbestrol, dinoprostone, dithranol, dutasteride, dexrazoxane, docetaxel, doxifluridine, erlotinib, estramustine, etoposide, exemestane, finasteride, flutamide, floxuridine, flucytosine, fludarabine, fluorouracil, ganciclovir, gefitinib, gemcitabine, goserelin, hydroxyurea, hydroxycarbamide, ifosfamide, irinotecan, imatinib, lenalidomide, leflunomide, letrozole, leuprorelin acetate, lomustine, mechlorethamine, melphalan, mercaptopurine, methotrexate, mitomycin, mitotane, menotropins, mifepristone, nafarelin, nelarabin, nitrogen mustard, nitrosoureas, oxaliplatin, ozogamicin, paclitaxel, podophyllyn, pegasparaginase, pemetrexed, pentamidine, pentostatin, procarbazin, raloxifene, ribavarin, raltitrexed, rituximab, romidepsin, sorafenib, streptozocin, sunitinib, sirolimus, streptozocin, temozolomide, temsirolimus, teniposide, thalidomide, thioguanine, thiotepa, topotecan, tacrolimus, taxotere, tafluposide, toremifene, tretinoin, trifluridine, triptorelin, valganciclovir, valrubicin, vinblastine, vidaradine, vincristine, vindesine, vinorelbine, vemurafenib, vismodegib, vorinostat, zidovudine, vedotine, derivatives and combinations thereof.

In a preferred aspect, the antibody-drug conjugates disclosed herein comprises a linker between the antibody and the drug. The linker disclosed herein may be cleavable or non-cleavable, preferably, the linker is cleavable. Examples of cleavable linkers disclosed herein include, without limitations, disulfides, hydrazones and peptides. Examples of non-cleavable linkers disclosed herein include, without limitations, thioethers.

In a particular aspect, the drug is linked to a cysteine or a lysine residue of the antibody. Preferably, the drug or antigen is linked to an unnatural amino acids that has been incorporated into the antibody.

Methods to make antibody-drug conjugates are well known from the man skilled in the art.

In another aspect, the disclosure concerns DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent as described above for use in the treatment of a cancer in a patient, which is not claimed.

Preferably, the cancer of the patient or a cancer sample from said patient presents level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs higher than their reference level.

Preferably, the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs are the percentage of these cells on the total number of cells, or on the total number of CAFs, in a cancer sample of said patient, and reference level(s) are reference percentage(s).

Thus, the disclosure concerns a DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent as described above for use in the treatment of a cancer in a patient wherein the cancer of the patient or a cancer sample from said patient presents percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs higher than their reference percentages.

The percentages are determined as described above, the reference percentages and the cancer are as defined above

The disclosure also concerns a pharmaceutical composition comprising a DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent, preferably a DPP4 inhibitor, a CD73 inhibitor, an anti-DPP4 antibody, an anti-OX40L antibody, an anti-CD73 antibody, an anti-PDL2 antibody, an anti-JAM2 antibody, an anti-B7H3 antibody or a combination thereof, which is not claimed, antibodies being optionally conjugated to a drug, preferably a cytotoxic drug, or a combination thereof, and at least one pharmaceutically acceptable excipient, for use in the treatment of a cancer in a patient. For this formulation, conventional excipient can be used according to techniques well known by those skilled in the art.

Preferably, the cancer of the patient or a cancer sample from said patient presents percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs, higher than their reference percentages. The percentages are determined as described above, the reference percentages and the cancer are as defined above.

The disclosure also relates to the use of a DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent according to the disclosure or of a pharmaceutical composition according to the disclosure, for the preparation of an anticancer medicament, which again is not claimed.

Preferably, the cancer of the patient or a cancer sample from said patient presents percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs higher than their reference percentages. The percentages are determined as described above, the reference percentages and the cancer are as defined above.

The DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent according to the disclosure or a pharmaceutical composition according to the disclosure may be to be administered by any convenient route. For instance, it can be to be administered by a systemic route, in particular by subcutaneous, intramuscular, intravenous or intradermal, preferably by intravenous, injection. It can also be to be directly administered in the inflammatory organ or tissue.

The DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent according to the invention or a pharmaceutical composition according to the invention may be to be administered as a single dose or in multiple doses.

The DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent according to the invention or a pharmaceutical composition according to the invention may be to be administered between every day and every month, preferably every week or every two weeks, more preferably every week.

The duration of treatment with a DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent according to the invention or with a pharmaceutical composition according to the invention is preferably comprised between 1 and 20 weeks, preferably between 1 and 10 weeks. Alternatively, the treatment may last as long as the cancer persists.

The amount of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent according to the invention or of a pharmaceutical composition according to the invention to be administered has to be determined by standard procedure well known by those of ordinary skills in the art. Physiological data of the patient (e.g. age, size, and weight) and the routes of administration have to be taken into account to determine the appropriate dosage, so as a therapeutically effective amount will be to be administered to the patient.

The disclosure also concerns a product or kit comprising a) a DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent according to the disclosure or a pharmaceutical composition according to the disclosure, and b) an immunotherapy treatment as described above, preferably an immune checkpoints inhibitor treatment as described above, as a combined preparation for simultaneous, separate or sequential use in the treatment of a cancer in a patient, which is not claimed.

In a product or kit according to the present disclosure, the active ingredients of the combined preparation can be to be administered simultaneously, separately or sequentially.

When the active ingredients of the combined preparation are to be administered separately or sequentially, especially when to be administered separately, the treatment with a DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent or with the pharmaceutical composition is preferably done before the immunotherapy treatment In that case, the product or kit for use may further comprise a step of determining the percentage of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs after the administration of the treatment with a DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent or with the pharmaceutical composition but prior the immunotherapy treatment, the immunotherapy treatment being administered only if the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs are lower than their reference percentage(s).

The active ingredients of the combined preparation can be to be administered to the subject by the same or different routes of administration. Administration routes usually depend on the pharmaceutical compositions used.

The form of the pharmaceutical compositions, the route of administration and the dose of administration of the DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs targeting agent, the pharmaceutical composition or the product or kit can be adjusted by the man skilled in the art according to the type and severity of the infection, and to the patient, in particular its age, weight, sex, and general physical condition.

### Prognosis methods

In another aspect, the present disclosure also relates to a method for predicting or monitoring clinical outcome of a subject affected with a cancer (not claimed) wherein the method comprises:
(a) detecting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient;
(b) determining the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample, wherein high level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs are predictive of a poor prognosis;
(c) optionally comparing the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample to reference level(s), wherein level(s) higher than their reference level(s) are predictive of a poor prognosis;
wherein a poor prognosis is preferably a poor survival prognosis, an early disease progression, an increased disease recurrence, especially after resection and/or treatment, and/or an increased metastasis occurrence.

Preferably, the method further comprise a step of providing a cancer sample from said patient.

The cancer and the patient are as described above.

The level of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs are determined as described above and the reference levels are as described above.

Preferably, the level(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample of a patient are the percentage(s) of these cells on the total number of cells, or on the total number of CAFs, in the cancer sample, and reference level(s) are reference percentage(s).

Thus, the present disclosure also relates to a method for predicting or monitoring clinical outcome of a subject affected with a cancer, wherein the method comprises:
(a) detecting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient;
(b) determining the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample, wherein high percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs are predictive of a poor prognosis.

The disclosure also relates to a method for predicting or monitoring clinical outcome of a subject affected with a cancer, wherein the method comprises:
(a) detecting DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in a cancer sample from said patient;
(b) determining the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample;
(c) comparing the percentage(s) of DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs in said cancer sample to reference percentage(s), wherein percentage(s) higher than their reference percentage(s) are predictive of a poor prognosis;
wherein a poor prognosis is preferably a poor survival prognosis, an early disease progression, an increased disease recurrence, especially after resection and/or treatment, and/or an increased metastasis occurrence.

Preferably, the method further comprise a step of providing a cancer sample from said patient.

The cancer and the patient are as described above. The DPP4⁺ CAFs, and/or CD73⁺ CAFs, and/or B7H3⁺ CAFs, and/or OX40L⁺ CAFs, and/or JAM2⁺ CAFs, and/or PDL2⁺ CAFs percentage(s) are determined as described above. The reference percentages are as described above.

### Kits

In another aspect, the disclosure also concerns the use of a kit for selecting a patient affected with a tumor for an immunotherapy treatment, preferably an immune checkpoint inhibitor treatment, and/or for predicting the efficacy of an immunotherapy treatment preferably an immune checkpoint inhibitor treatment, wherein the kit comprises detection means selected from the group consisting of a pair of primers, a probe and an antibody specific to a protein selected from the group consisting of DPP4, OX40L, CD73, PDL2, JAM2, B7H3, and a combination thereof, and, optionally, a leaflet providing guidelines to use such a kit. None of these aspects are claimed.

Although having different meanings, the terms "comprising", "having", "consisting in" and "containing" can be replaced one for the other in the entire application.

Further aspects and advantages of the present disclosure will be described in the following examples, which should be regarded as illustrative and not limiting.

### Examples

### Example 1:

### Materials and Methods

### Silencing experiments using small-interfering RNA (siRNA)

For functional assays, primary cultures of CAFs were transfected with a pool of 5 specific siRNA: siTNFSF4/OX40L (#GS7292), siPDCD1LG2/PD-L2 (#GS80380), siCD276B7H3 (#GS80381), siNT5E/CD73 (#GS4907) and siDPP4 (#GS1803) or non-targeting siRNA (siCTR, AllStarts negative control, #1027281). All siRNAs (FlexiTube Gene Solution siRNA #1027416) and non-targeting siCTR were from Qiagen. Transfections were carried out at a final concentration of 20 nM using DharmaFECT 1 (Dharmacon, #T-2001-02) transfection reagent according to manufacturer's instructions.

### Isolation of CD4⁺CD25⁺ T cells

Isolation of CD4⁺CD25⁺ T cells: CD4⁺CD25⁺ T-lymphocytes were isolated from peripheral blood of healthy donors obtained from the "Etablissement Français du Sang", Paris, Saint-Antoine Crozatier blood bank through a convention with the Institut Curie (Paris, France). Briefly, peripheral blood mononuclear cells (PBMCs) were isolated using Lymphoprep (STEMCELL, #07861) as previously described and 500 million PBMCs were used for CD4⁺CD25⁺ purification by magnetic cell separation (MACS) with the Human CD4⁺CD25⁺ T-regs Isolation kit (Miltenyi Biotec, #130-091-301) according to manufacturer's instructions. The purity of CD4⁺CD25⁺ T cells purified was determined by flow cytometry.

### Co-culture of T cells with CAFs time-lapse video microscopy

For co-culture experiments for time-lapse video microscopy, CAFs (50-60 000 cells) were transiently transfected with siRNA (listed above) and plated in 12-well plates in DMEM supplemented with 10% FBS. Purified CD4⁺CD25⁺ T-lymphocytes were freshly added to CAF cells, 30h post-transfection, to reach a ratio of approximately 1:5 (CAF:T-cell). Just before adding the T cells, the media was replaced to DMEM supplemented with 2.5% FBS and immediately placed under a conditioned chamber (37°C) of a Leica video-microscope and recorded for 28 h. Microphotographs were captured in 5 different representative positions every 8 minutes for each well, resulting in 5 videos per experimental condition generated using Metamorph software.

### Analysis of the time-lapse videos

An automatic pipeline, which combines ImageJ plugins and R scripts, was developed to define co-localization between CAFs and CD4⁺CD25⁺ T-lymphocytes and follow cell trajectories over time. Normalization was performed by removing median background of all videos. Area of CAF was then evaluated using the ImageJ plugin Phantast (https://github.com/nicjac/PHANTAST-FIJI) with the following parameters: Sigma=1 and Epsilon=0.03. ImageJ plugin Particle Tracker (http://imagej.net/Particle_Tracker) from the MOSAIC ToolSuite (http://mosaic.mpi-cbg.de/?q=downloads/imageJ) was used to detect automatically CD4⁺CD25⁺ T-lymphocytes and track immune cells trajectories throughout the video. The following parameters were used: Radius=3, Cutoff=3, Per/Abs=0.1, Link Range=10, Displacement=10. Finally, the exact position of both CAF and T cells was determined and combined for each time-frame allowing detection of co-localization of the two cell types. Two types of interactions between CAF and T cells were considered based on two parameters, the number of time frames (bin) and the number of minimal contacts (minC) within this time frame window. For each T-lymphocyte analyzed, persistent interaction with CAFs was defined as at least 8 contacts observed during a time window of 14 frames (bin=14 and minC=8). All interactions, including short and persistent contacts, were quantified considering at least 1 contact observed during a time window of 10 frames (bin=10 and minC=1). 4 Videos corresponding to 28h (210 frames, 8 min per frame) were analyzed for each condition.

### Co-culture of T cells with CAFs and FOXP3 induction

To study the impact of CAFs on CD4⁺CD25⁺ T cells, co-cultures were performed. 50 000 primary CAFs were plated in 24-well plates in DMEM supplemented with 10% FBS and used non-transfected or siRNA-transfected (siCTR, siCD276/B7H3, siNT5E/CD73, siDPP4 and siTNFSF4/OX40L). The medium was replaced by fresh DMEM supplemented with 1% FBS just before 500 000 CD4⁺CD25⁺ T-lymphocytes were added to CAFs, or to CAFs + siRNA 30h post-transfection. Co-cultures of CAFs and CD4⁺CD25⁺ T cells were incubated for 16 h at 37°C, 3% O2. Negative control was incubation of CD4⁺CD25⁺ T cells without CAFs. After incubation, T cells were collected and analyzed by FACS. In brief, T-lymphocytes were incubated with LIVE/DEAD (Thermo Fisher Scientific) for 20 min at RT for exclusion of dead cells. After incubation, cells were washed in PBS+ for 10 min and incubated with an antibody cocktail in PBS+ containing anti-CD45-APC-Cy7, anti-CD3-Alexa Fluor 700 (BD Biosciences, #557943), anti-CD4-APC (Miltenyi Biotec, #130-092-374) and anti-CD25-PE (Miltenyi Biotec, #130-091-024) for 15 min at RT. The detection of FOXP3 was performed using the FOXP3 staining buffer set kit (eBioscience) for fixation and permeabilization according to manufacturer's instructions followed by incubation with anti-Foxp3-Alexa Fluor 488 (eBioscience, #53-4776) for 30 min at RT. For CD25 and FOXP3 staining, the corresponding isotype controls were (#130-092-215 and #53-4321 for CD25 and FOXP3, respectively). Analyses were performed in the BD LSR II flow cytometer (BD Biosciences) and data was then analyzed using FlowJo version 9.8.1.

### Results

To investigate the dynamic interaction between CAF fibroblasts and CD4⁺CD25⁺ T-lymphocytes, the inventors performed time-lapse video-microscopy during 48 h of co-culture of the two cell types (Fig. 1a-d). They first detected a close proximity between CAF cells and CD4⁺CD25⁺ T-lymphocytes (Fig. 1a), confirming the capacity of CAF cells to attract T-cells. Two types of interaction were observed between CAF fibroblasts and T cells: a short-time contact ("come and go") occurring during a short period of time (Fig. 1a, long arrows) and a persistent interaction of at least 2 h (Fig. 1a, short arrows). An automated tool was developed enabling to define the co-localization of CAFs and immune cells and to determine cell trajectories over time. Interactions between immune and CAF cells over time were analyzed and expressed using Kaplan-Meier curves, where each event represented a loss of contact between the two cell types (Fig. 1b, c). Using this method, it was determined that the minimum median time of persistent interactions between CAF fibroblasts and CD4⁺CD25⁺ T-lymphocytes can reach 14 h (Fig. 1b Left). Moreover, the frequency of persistent interactions between CAFs and T-cells accounted for 20% of total contacts (Fig. 1b, Right). Among the genes highly expressed by CAF fibroblasts, the inventors identified the ligands TNFSF4/OX40L and PDCD1LG2/PD-L2, as key players in these long-term interactions between stromal and immune cells (Fig. 1c). Indeed, it was observed that the median time of interaction between CAFs and T-lymphocytes dropped significantly, upon silencing of these molecules in CAF cells (Fig. 1c and Fig. 2 for efficiency of silencing). The frequency of persistent contacts between CAFs and T-cells (around 20% of total contacts) remained equivalent in all conditions (Fig. 1d, Left). However, the duration of retention of T-lymphocytes was shortened by the silencing of either OX40L or PD-L2 in CAF fibroblasts (Fig. 5d, Right), thus indicating that CAF fibroblasts retain CD4⁺CD25⁺ T-lymphocytes at their surface through at least OX40L and PD-L2.

Finally, the inventors also demonstrated that CAF stromal cells could also enhance the survival and the differentiation of T-lymphocytes into CD25⁺FOXP3⁺ T-regs. Indeed, CAF cells increased the total number of CD25⁺FOXP3⁺ T-cells and enhanced their survival. Moreover, the increase in total CD25⁺FOXP3⁺ T-cells in presence of CAF cells was independent of their pro-survival role, suggesting that CAF cells not only enhance T-regs survival but also promote their differentiation. Importantly, the silencing of CD276/B7H3, NT5E/CD73 or DPP4 in CAF fibroblasts significantly reduced the impact of CAF fibroblasts on the total number of CD25⁺FOXP3⁺ T-cells, while inactivation of OX40L had no impact (Fig. 1f). Moreover, the impact of CD73 and DPP4 on CD25⁺FOXP3⁺ T-cells activation was strictly observed in the FOXP3high population, while B7H3 had a broader effect by acting on both FOXP3med and FOXP3high populations (Fig. 1e-g).

### Example 2:

### Materials and Methods

### Silencing experiments using small-interfering RNA (siRNA)

For functional assays, primary cultures of CAFs were transfected with a pool of 2 specific siRNA: siCD276/B7H3 (#GS80381) and siNT5E/CD73 (#GS4907) or non-targeting siRNA (siCTR, AllStarts negative control, #1027281). All siRNAs (FlexiTube Gene Solution siRNA #1027416) and non-targeting siCTR were from Qiagen. Transfections were carried out at a final concentration of 20 nM using DharmaFECT 1 (Dharmacon, #T-2001-02) transfection reagent according to manufacturer's instructions.

### Isolation of CD4⁺CD25⁺ T cells

CD4⁺CD25⁺ T-lymphocytes were isolated from peripheral blood of healthy donors obtained from the "Etablissement Français du Sang", Paris, Saint-Antoine Crozatier blood bank through a convention with the Institut Curie (Paris, France). Briefly, peripheral blood mononuclear cells (PBMCs) were isolated using Lymphoprep (STEMCELL, #07861) as previously described and 500 million PBMCs were used for CD4⁺CD25⁺ purification by magnetic cell separation (MACS) with the Human CD4⁺CD25⁺ T-regs Isolation kit (Miltenyi Biotec, #130-091-301) according to manufacturer's instructions. The purity of CD4⁺CD25⁺ T cells purified was determined by flow cytometry.

### Co-culture of T cells with CAFs and FOXP3 induction

To study the impact of CAF on CD4⁺CD25⁺ T cells, co-cultures were performed. 50 000 primary CAFs were plated in 24-well plates in DMEM supplemented with 10% FBS and used non-transfected or siRNA-transfected (siCTR, siCD276/B7H3, siNT5E/CD73, siDPP4 and siTNFSF4/OX40L). The medium was replaced by fresh DMEM supplemented with 1% FBS just before 500 000 CD4⁺CD25⁺ T-lymphocytes were added to CAF, or to CAF + siRNA 30h post-transfection. Co-cultures of CAFs and CD4⁺CD25⁺ T cells were incubated for 16 h at 37°C, 3% O2. Negative control was incubation of CD4⁺CD25⁺ T cells without CAFs. After incubation, T cells were collected and analyzed by FACS. In brief, T-lymphocytes were incubated with LIVE/DEAD (Thermo Fisher Scientific) for 20 min at RT for exclusion of dead cells. After incubation, cells were washed in PBS+ for 10 min and incubated with an antibody cocktail in PBS+ containing anti-CD45-APC-Cy7, anti-CD3-Alexa Fluor 700 (BD Biosciences, #557943), anti-CD4-APC (Miltenyi Biotec, #130-092-374) and anti-CD25-PE (Miltenyi Biotec, #130-091-024) for 15 min at RT. The detection of FOXP3 was performed using the FOXP3 staining buffer set kit (eBioscience) for fixation and permeabilization according to manufacturer's instructions followed by incubation with anti-Foxp3-Alexa Fluor 488 (eBioscience, #53-4776) for 30 min at RT. For CD25 and FOXP3 staining, the corresponding isotype controls were (#130-092-215 and #53-4321 for CD25 and FOXP3, respectively). Analyses were performed in the BD LSR II flow cytometer (BD Biosciences) and data was then analyzed using FlowJo version 9.8.1.

### Results

The inventors found that CAF stromal cells from HGSOC could also, as in breast cancer, enhance the survival and the differentiation of T-lymphocytes into CD25⁺FOXP3⁺ T-regs. Indeed, CAF cells increased the total number of CD25⁺FOXP3⁺ T-cells and enhanced their survival. Moreover, the increase in total CD25⁺FOXP3⁺ T-cells in presence of CAF cells was independent of their pro-survival role, suggesting CAF cells not only enhance T-regs survival but also promote their differentiation. Importantly, the silencing of CD276/B7H3 and NT5E/CD73 in CAF fibroblasts significantly reduced the impact of CAF fibroblasts on the total number of CD25⁺FOXP3⁺ T-cells. The impact of CD73 and NT5E on CD25⁺FOXP3⁺ T-cells activation was observed in the FOXP3high population.

### Example 3: Computation of the percentage of CAFs cells positive for DPP4, OX40L, CD73, PDL2, and B7H3

### Materials and Methods

Single-cell RNA-Seq was performed on 96 CAFs coming from 2 Breast Cancer patients (subtype Lum A).

Fresh human breast tumor samples were cut into small pieces and an enzymatic digestion was performed in CO₂ independent medium (Gibco #18045-054) using 150ug/mL Liberase (Roche, #05401020001), and 150µg/mL DNase I (Roche #11284932001) during 30 minutes in a 37°C incubator with agitation (180rpm). Cells were then filtrated with a 40µm cell strainer (Fisher Scientific #223635447) and resuspended in PBS+ (PBS, Gibco #14190; EDTA 2mM, Gibco #15575; Human Serum 1%, BioWest # S4190-100). Cells were stained using a pool of primary antibodies 15min at room temperature in PBS+. 3mM DAPI (ThermoFisher scientific #D1306) was added just before flow cytometry analysis. Cell suspensions were sorted on BD FACS Aria.

CAF cells were collected after FACS sorting in RNAase free tubes previously coated with CO₂ independent medium supplemented with 15% serum. They were obtained from two fresh breast cancer samples, each experiment (digestion, sorting, capture and cDNA synthesis) being done on different days.

Cells were diluted with Fluidigm suspension reagent (4:1) and then directly loaded on a primed 17-25 um C1 Single-Cell auto prep array for mRNAseq (Fluidigm #100-5761). Cell capture, lysis, reverse transcription and cDNA amplification were done on a Fluidigm C1 with SMARTer Ultra Low input RNA kit for the Fluidigm C1 System (Clonetech, #634833, Fluidigm #100-6201) according to the manufacturers protocols. Single cell capture was manually checked with a Leica microscope, cDNA quality was checked on a LabChip GX (PerkinElmer) and only good quality cDNA from single cells were further processed to prepare cDNA library using Nextera XT preparation kit (Illumina). Sequencing and further analyses were done on all data, pooling single cells from the two tumors. Samples were sequenced on a rapid run flow cell of HiSeq 2500 (Illumina) with an average sequencing depth of 9 million of paired-end reads. Length of the reads was 100bp. Reads were mapped on reference human genome (hg19/GRCh37 from UCSC genome release) using Tophat _2.0.6 algorithm with the following parameters: global alignment, no mismatch in seed alignment (of size 22), 3 mismatches in read length. Quality control was performed using FastQC software. Quantification of expression at both gene and transcript level was done using HTSeq-count and featureCouts (implemented in Bioconductor R package Rsubread). Only genes with at least one read in at least one sample were kept for further analyses. Normalization was performed using method implemented in DESeq2 R package.

Percentage of positive cells was calculated for each gene as the ratio of the number of cells with normalized log-expression higher than 3 to the total number of cells.

### Results

Based on the single-cell RNA-Seq data (cf. Figure 4), the percentage of DPP4⁺, OX40L⁺, CD73⁺, PDL2⁺, and B7H3⁺ cells were established.
Results obtained were the following: DPP4⁺ CAFs: 19 cells among 96 with DPP4 expression higher than 3, i.e. 20%, OX40L⁺ CAFs; 17 cells among 96 with OX40L expression higher than 3, i.e. 18%, CD73⁺ CAFs: 46 cells among 96 with NT5E expression higher than 3, i.e. 48%, PDL2⁺ CAFs: 9 cells among 96 with PDCD1LG2 expression higher than 3, i.e. 9% and B7H3⁺ CAFs: 31 cells among 96 with CD276 expression higher than 3, i.e. 32%.

### Example 4:

### Materials and Methods

RNA-Seq data of 28 pre-treatment (pembrolizumab and nivolumab) melanoma tumors (responding, n=15; non-responding, n=13) were retrieved from Gene Expression Omnibus (GEO) under accession number GSE78220¹. Responding patients included complete or partial responses to anti-PD-1 therapy while non-responding patients was defined by patients who had progressive disease.

CAF-S1 and CAF-S4 signatures were retrieved from Costa et al. ². The top 15 genes up-regulated in CAF-S1 and up-regulated in CAF-S4 were selected and the geometric mean was computed and used to compare responders and non-responder. Based on single-cell RNA-Seq data obtained on 3000 CAF-S1 cells using 10X Genomics Chromium technology, 6 sub-populations of CAF-S1 (named sub-populations 0 to 5) were identified (manuscript in preparation). A score was established for each CAF-S1 cluster by calculating the geometric mean of the top 20 genes specifically up-regulated in each cluster.

Differential expression between responder and non-responder was assessed using Welch two-sample t-test. P-value < 0.05 was considered as significant.

### Results

Transcriptomic data of 28 pre-treatment melanomas undergoing anti-PD-1 checkpoint inhibition therapy¹ were analyzed. Differences between non-responding (n=13) and responding (n=15) patients were assessed for genes that characterize CAF-mediated immunosuppression as well as for genes encoding for PD-1, PD-L1, PD-L2 and genes involved in cytotoxic immune response (Figure 5). Interestingly, only CAF markers showed a significant over-expression in non-responding patients (FAP: p=0.04; DPP4: p=0.03; JAM2: p=0.01). It was investigated if the two activated CAF sub-populations (CAF-S1 and CAF-S4) described in Costa et al.² could be predictive of the response to immunotherapy. Results showed that only gene signature representative of the immunosuppressive CAF-S1 subset was predictive of immunotherapy response while no differences between responding and non-responding tumors were observed in CAF-S4 subset. To go further, heterogeneity within the immunosuppressive CAF-S1 subset was assessed by single-cell RNA-Seq and six clusters were identified (called sub-population 0 to sub-population 5). Results showed that 2 sub-populations among the 6 were significantly predictive of the response to immunotherapy, the sub-population 1 and 3 (Figure 6). Since the markers of CAFS1 and 4 are present in the most aggressive tumors and in patients resistant to immunotherapy, these markers should be ipso facto related to a poor prognosis.

### Reference

1- Hugo W, Zaretsky JM, Sun L, Song C, Moreno BH, Hu-Lieskovan S, Berent-Maoz B, Pang J, Chmielowski B, Cherry G, Seja E, Lomeli S, Kong X, Kelley MC, Sosman JA, Johnson DB, Ribas A, Lo RS. Genomic and Transcriptomic Features of Response to Anti-PD-1 Therapy in Metastatic Melanoma. Cell. 2016 Mar 24;165(1):35-44.
2- Costa A, Kieffer Y, Scholer-Dahirel A, Pelon F, Bourachot B, Cardon M, Sirven P, Magagna I, Fuhrmann L, Bernard C, Bonneau C, Kondratova M, Kuperstein I, Zinovyev A, Givel AM, Parrini MC, Soumelis V, Vincent-Salomon A, Mechta-Grigoriou F. Fibroblast Heterogeneity and Immunosuppressive Environment in Human Breast Cancer. Cancer Cell. 2018 Mar 12;33(3):463-479.e10

## Claims

1. An *in vitro* method for selecting a patient affected with a solid tumor for an immune checkpoint inhibitor treatment, wherein the method comprises:
(a) detecting DPP4⁺ CAFs (Cancer Associated Fibroblast) in a cancer sample from said patient;
(b) determining the level of DPP4⁺ CAFs in said cancer sample, wherein the responsiveness of said patient to an immune checkpoint inhibitor treatment is inversely proportional to the level of DPP4⁺ CAFs in the cancer sample;
(c) selecting patients with level of DPP4⁺ CAFs lower than their reference level as suitable for an immune checkpoint inhibitor treatment, and/or selecting patients with a level of DPP4⁺ CAFs higher than their reference level for a cancer treatment excluding immune checkpoint inhibitor.

2. An *in vitro* method for predicting the response of a subject affected with a solid tumor to an immune checkpoint inhibitor treatment, wherein the method comprises:
(a) detecting DPP4⁺ CAFs (Cancer Associated Fibroblast) in a cancer sample from said patient;
(b) determining the level of DPP4⁺ CAFs in said cancer sample, wherein the responsiveness of said patient to an immune checkpoint inhibitor treatment is inversely proportional to the level of DPP4⁺ CAFs in the cancer sample;
(c) comparing the level of DPP4⁺ CAFs in said cancer sample to reference level, wherein level lower than their reference level is predictive of the responsiveness of said patient to an immune checkpoint inhibitor treatment, and/or level higher than their reference level is predictive of the inefficacy or lower efficacy of an immune checkpoint inhibitor treatment on the cancer of said patient.

3. The method according to claim 1, wherein the method further comprises:
(a) detecting CD73+ CAFs, and/or B7H3+ CAFs, and/or OX40L+ CAFs, and/or JAM2+ CAFs in the cancer sample from said patient, and
(b) determining the level of CD73+ CAFs, and/or B7H3+ CAFs, and/or OX40L+ CAFs, and/or JAM2+ CAFs in said cancer sample, wherein the responsiveness of said patient to an immune checkpoint inhibitor treatment is inversely proportional to the levels of DPP4+ CAFs, and: CD73+ CAFs, and/or B7H3+ CAFs, and/or OX40L+ CAFs, and/or JAM2+ CAFs in the cancer sample,
(c) selecting patients with level of DPP4⁺ CAFs and: CD73+ CAFs, and/or B7H3+ CAFs, and/or OX40L+ CAFs, and/or JAM2+ CAFs lower than their reference level as suitable for an immune checkpoint inhibitor treatment, and/or selecting patients with a level of DPP4⁺ CAFs and: CD73+ CAFs, and/or B7H3+ CAFs, and/or OX40L+ CAFs, and/or JAM2+ CAFs higher than their reference level for a cancer treatment excluding immune checkpoint inhibitor.

4. An immune checkpoint inhibitor for use in the treatment of a solid cancer in a patient wherein the patient has been selected for treatment by a method according to claim 1 or 3, and wherein the patient presents in a cancer sample:
(a) no DPP4⁺ CAFs; or
(b) level of DPP4⁺ CAFs lower than their reference level.

5. The immune checkpoint inhibitor for use according to claim 4, wherein the patient further presents in a cancer sample:
(a) no CD73+ CAFs, and/or no B7H3+ CAFs, and/or no OX40L+ CAFs, and/or no JAM2+ CAFs, or
(b) level(s) of CD73+ CAFs, and/or B7H3+ CAFs, and/or OX40L+ CAFs, and/or JAM2+ CAFs lower than their reference level.

6. The method according to any one of claims 1-3 or the immune checkpoint inhibitor for use according to claim 4 or 5, wherein the checkpoint inhibitor is selected from the group consisting of an anti-CTLA-4 (cytotoxic T lymphocyte associated protein 4) therapies, PD-1 (programmed cell death protein 1) inhibitors, PDL1 (programmed cell death ligand) inhibitors, LAG-3 (Lymphocyte-activation gene 3) inhibitors, TIM-3 (T-cell immunoglobulin and mucin-domain containing-3) inhibitors, TIGIT (T cell immunoreceptor with Ig and ITIM domains) inhibitors, BLTA (B- and T-lymphocyte attenuator) inhibitors, IDO1 inhibitors, or a combination thereof.

7. The method or the immune checkpoint inhibitor for use according to claim 6, wherein the immune checkpoint inhibitor is a PD-1 (programmed cell death protein 1) inhibitor or a PDL1 (programmed cell death ligand) inhibitor.

8. The method or the immune checkpoint inhibitor for use according to claim 6 or 7, wherein the anti-CTLA-4 therapy is ipilimumab, the PD-1 inhibitor is nivolumab, pembrolizumab, or BGB-A317, the PDL1 inhibitor is atezolizumab, avelumab, or durvalumab, the LAG-3 inhibitor is BMS-986016, the IDO1 inhibitor is epacadostat.

9. The method for selecting a patient according to anyone of claims 1-3 or 6-8, or the immune checkpoint inhibitor for use according to anyone of claims 4-8, wherein the level of DPP4⁺ CAFs are the percentage of DPP4⁺ CAFs in the cancer sample and the reference level are reference percentage.

10. The method for selecting a patient according to anyone of claims 1-3 or 6-8, or the immune checkpoint inhibitor for use according to anyone of claims 4-8, wherein the percentage of DPP4⁺ CAFs in the cancer sample is calculated as the percentage DPP4⁺ CAFs cells on the total number of cells in the cancer sample.

11. The method for selecting a patient according to anyone of claims 1-3 or 6-10, or the immune checkpoint inhibitor for use according to anyone of claims 4-10, wherein the solid cancer is selected from the group consisting of prostate cancer, lung cancer, breast cancer, gastric cancer, kidney cancer, ovarian cancer, hepatocellular cancer, osteosarcoma, melanoma, hypopharynx cancer, esophageal cancer, endometrial cancer, cervical cancer, pancreatic cancer, liver cancer, colon or colorectal cancer, neuroendocrine tumors, muscle cancer, adrenal cancer, thyroid cancer, uterine cancer, skin cancer, bladder cancer, head and neck cancer, pediatric cancer.

12. The method for selecting a patient according to anyone of claims 1-3 or 6-10, or the immune checkpoint inhibitor for use according to anyone of claims 4-10, wherein the solid cancer is selected from the group consisting of ovarian cancer, breast cancer, lung cancer, colorectal cancer, pancreatic cancer and pediatric cancer.

13. The method for selecting a patient according to anyone of claims 1-3 or 6-10, or the immune checkpoint inhibitor for use according to anyone of claims 4-10, wherein the cancer is an ovarian cancer, preferably a mesenchymal ovarian cancer, in particular high grade ovarian cancer of the serous type.

14. The method for selecting a patient according to anyone of claims 1-3 or 6-10, or the immune checkpoint inhibitor for use according to anyone of claims 4-10, wherein the cancer is a breast cancer, preferably an invasive breast cancer and/or its metastasis, in particular axillary metastasis.

## Patentansprüche

1. In-vitro-Verfahren zur Auswahl eines Patienten mit einem soliden Tumor für eine Immun-Checkpoint-Inhibitor-Behandlung, wobei das Verfahren Folgendes umfasst:
(a) Nachweis von DPP4⁺ CAFs (Cancer Associated Fibroblast) in einer Krebsprobe von diesem Patienten;
(b) Bestimmen des Gehalts an DPP4⁺ CAFs in dieser Krebsprobe, wobei das Reagieren dieses Patienten auf eine Immun-Checkpoint-Inhibitor-Behandlung umgekehrt proportional zum Gehalt an DPP4⁺ CAFs in der Krebsprobe ist:
(c) Auswahl von Patienten mit einem Gehalt an DPP4⁺ CAFs unter ihrem Referenzwert als geeignet für eine Immun-Checkpoint-Inhibitor-Behandlung und/oder Auswahl von Patienten mit einem Gehalt an DPP4⁺ CAFs über ihrem Referenzwert für eine Krebsbehandlung ohne Immun-Checkpoint-Inhibitor

2. *In-vitro*-Verfahren zur Vorhersage des Ansprechens eines Patienten mit einem soliden Tumor auf eine Immun-Checkpoint-Inhibitor-Behandlung, wobei das Verfahren Folgendes umfasst:
(a) Nachweis von DPP4⁺ CAFs (Cancer Associated Fibroblast) in einer Krebsprobe von diesem Patienten;
(b) Bestimmen des Gehalts an DPP4⁺ CAFs in dieser Krebsprobe, wobei das Reagieren dieses Patienten auf eine Immun-Checkpoint-Inhibitor-Behandlung umgekehrt proportional zum Gehalt an DPP4+-CAFs in der Krebsprobe ist:
(c) Vergleichen des Gehalts an DPP4⁺ CAFs in dieser Krebsprobe mit dem Referenzwert, wobei ein Gehalt unter ihrem Referenzwert ein Reagieren dieses Patienten auf eine Immun-Checkpoint-Inhibitor-Behandlung vorhersagen kann und/oder ein Gehalt über ihrem Referenzwert die Unwirksamkeit oder geringere Wirksamkeit einer Immun-Checkpoint-Inhibitor-Behandlung bei der Krebserkrankung dieses Patienten vorhersagen kann.

3. Verfahren nach Anspruch 1, wobei das Verfahren weiterhin umfasst:
(a) Nachweis von CD73⁺ CAFs und/oder B7H3⁺ CAFs und/oder OX40L⁺ CAFs und/oder JAM2⁺ CAFs in der Krebsprobe von diesem Patienten und
(b) Bestimmen des Gehalts an CD73⁺ CAFs und/oder B7H3⁺ CAFs und/oder OX40L⁺ CAFs und/oder JAM2⁺ CAFs in dieser Krebsprobe, wobei das Reagieren des Patienten auf eine Immun-Checkpoint-Inhibitor-Behandlung umgekehrt proportional zu den Gehalten an DPP4⁺ CAFs und: CD73⁺ CAFs und/oder B7H3⁺ CAFs und/oder OX40L⁺ CAFs und/oder JAM2+ CAFs in der Krebsprobe ist,
(c) Auswahl von Patienten mit Gehalt an DPP4⁺ CAFs und: CD73⁺ CAF und/oder B7H3⁺ CAF und/oder OX40L⁺ CAF und/oder JAM2⁺ CAF unter ihrem Referenzwert als geeignet für eine Immun-Checkpoint-Inhibitor-Behandlung und/oder Auswahl von Patienten mit Gehalt an DPP4⁺ CAFs und: CD73⁺ CAFs und/oder B7H3⁺ CAFs und/oder OX40L⁺ CAFs und/oder JAM2⁺ CAFs über ihrem Referenzwert für eine Krebsbehandlung ohne Immun-Checkpoint-Inhibitor.

4. Immun-Checkpoint-Inhibitor zur Verwendung bei der Behandlung eines soliden Krebses bei einem Patienten, wobei der Patient für die Behandlung nach einem Verfahren gemäß Anspruch 1 oder 3 ausgewählt wurde und wobei der Patient in einer Krebsprobe zeigt:
(a) keine DPP4⁺ CAFs; oder
(b) eine Konzentration von DPP4⁺ CAFs, die unter dem Referenzwert liegen.

5. Der Immun-Checkpoint-Inhibitor zur Verwendung nach Anspruch 4, wobei der Patient weiter in einer Krebsprobe zeigt:
(a) keine CD73+ CAFs und/oder keine B7H3+ CAFs und/oder keine OX40L+ CAFs und/oder keine JAM2+ CAFs, oder
(b) Konzentration(en) von CD73+-CAFs und/oder B7H3+-CAFs und/oder OX40L+-CAFs und/oder JAM2+-CAFs, die unter ihrem Referenzwert liegen.

6. Das Verfahren nach einem der Ansprüche 1-3 oder die Immun-Checkpoint-Inhibitor-Behandlung zur Verwendung nach Anspruch 4 oder 5, wobei die Checkpoint-Inhibitor ausgewählt ist aus der Gruppe bestehend aus Anti-CTLA-4 (zytotoxisches T-Lymphozytenassoziiertes Protein 4) Therapien, PD-1- (programmiertes Zelltodprotein 1) -Inhibitoren, PDL1(programmierter Zelltodligand)-Inhibitoren, LAG-3(Lymphozytenaktivierungsgen 3) - Inhibitoren, TIM-3(T-Zell-Immunglobulin und Muzin-Domäne enthaltende-3) -Inhibitoren, TIGIT(T-Zell-Immunrezeptor mit Ig- und ITIM-Domänen) -Inhibitoren, BLTA(B- und T-Lymphozyten-Attenuator) -Inhibitoren, IDO1-Inhibitoren oder eine Kombination davon.

7. Das Verfahren oder der Immun-Checkpoint-Inhibitor zur Verwendung nach Anspruch 6, wobei die Immun-Checkpoint-Inhibitor ein PD-1(programmiertes Zelltodprotein I) -Inhibitor oder ein PDL-1(programmierter Zelltodligand) -Inhibitor ist.

8. Das Verfahren oder der Immun-Checkpoint-Inhibitor zur Verwendung nach Anspruch 6 oder 7, wobei die Anti-CTLA-4-Therapie Ipilimumab, der PD-1-Inhibitor Nivolumab, Pembrolizumab oder BGB-A317 ist, der PDL1-Inhibitor Atezolizumab, Avelumab oder Durvalumab ist, der LAG-3-Inhibitor BMS-986016 ist, der IDOI-Inhibitor Epacadostat ist.

9. Das Verfahren zur Auswahl eines Patienten nach einem der Ansprüche 1-3 oder 6-8 oder der Immun-Checkpoint-Inhibitor zur Verwendung nach einem der Ansprüche 4-8, wobei die Menge an DPP4⁺ CAFs der Prozentsatz der DPP4⁺ CAFs in der Krebsprobe und der Referenzwert Referenzprozentsätze sind.

10. Das Verfahren zur Auswahl eines Patienten nach einem der Ansprüche 1-3 oder 6-8 oder der Immun-Checkpoint-Inhibitor zur Verwendung nach einem der Ansprüche 4-8, wobei der Prozentsatz an DPP4⁺ CAFs in der Krebsprobe berechnet wird als der prozentuale Anteil der DPP4⁺ CAFs-Zellen an der Gesamtzahl der Zellen in der Krebsprobe.

11. Das Verfahren zur Auswahl eines Patienten nach einem der Ansprüche 1-3 oder 6-10 oder der Immun-Checkpoint-Inhibitor zur Verwendung nach einem der Ansprüche 4-10, wobei der solide Krebs ausgewählt ist aus der Gruppe bestehend aus Prostatakrebs, Lungenkrebs, Brustkrebs, Magenkrebs, Nierenkrebs, Eierstockkrebs, Leberzellkrebs, Osteosarkom, Melanom, Hypopharynxkrebs, Speiseröhrenkrebs, Endometriumkrebs, Gebärmutterhalskrebs, Bauchspeicheldrüsenkrebs, Leberkrebs, Dickdarm- oder Darmkrebs, neuroendokrinen Tumoren, Muskelkrebs, Nebennierenkrebs, Schilddrüsenkrebs, Gebärmutterkrebs, Hautkrebs, Blasenkrebs, Kopf- und Nackenkrebs, Kinderkrebs.

12. Das Verfahren zur Auswahl eines Patienten nach einem der Ansprüche 1-3 oder 6-10 oder der Immun-Checkpoint-Inhibitor zur Verwendung nach einem der Ansprüche 4-10, wobei der solide Krebs ausgewählt ist aus der Gruppe bestehend aus Eierstockkrebs, Brustkrebs, Lungenkrebs, Darmkrebs, Bauchspeicheldrüsenkrebs und Kinderkrebs.

13. Das Verfahren zur Auswahl eines Patienten nach einem der Ansprüche 1-3 oder 6-10 oder der Immun-Checkpoint-Inhibitor zur Verwendung nach einem der Ansprüche 4-10, wobei der Krebs ein Eierstockkrebs ist, vorzugsweise ein mesenchymaler Eierstockkrebs, insbesondere hochgradiger Eierstockkrebs des serösen Typs.

14. Das Verfahren zur Auswahl eines Patienten nach einem der Ansprüche 1-3 oder 6-10 oder der Immun-Checkpoint-Inhibitor zur Verwendung nach einem der Ansprüche 4-10, wobei der Krebs ein Brustkrebs ist, vorzugsweise invasiver Brustkrebs und/oder dessen Metastasierung, insbesondere axilläre Metastasierung.

## Revendications

1. Méthode *in vitro* de sélection d'un patient atteint d'une tumeur solide pour un traitement par inhibiteur de point de contrôle immunitaire, ladite méthode comprenant :
(a) la détection de CAF (fibroblaste associé au cancer) DPP4⁺ dans un échantillon cancéreux provenant dudit patient ;
(b) la détermination du taux de CAF DPP4⁺ dans ledit échantillon cancéreux, la capacité de réponse dudit patient à un traitement par inhibiteur de point de contrôle immunitaire étant inversement proportionnelle au taux de CAF DPP4⁺ dans l'échantillon cancéreux ;
(c) la sélection de patients ayant un taux de CAF DPP4⁺ inférieur à leur taux de référence comme approprié pour un traitement par inhibiteur de point de contrôle immunitaire, et/ou la sélection de patients ayant un taux de CAF DPP4⁺ supérieur à leur taux de référence pour un traitement du cancer à l'exclusion de l'inhibiteur de point de contrôle immunitaire.

2. Méthode *in vitro* de prédiction de la réponse d'un sujet atteint d'une tumeur solide à un traitement par inhibiteur de point de contrôle immunitaire, ladite méthode comprenant :
(a) la détection de CAF (fibroblaste associé au cancer) DPP4⁺ dans un échantillon cancéreux provenant dudit patient ;
(b) la détermination du taux de CAF DPP4⁺ dans ledit échantillon cancéreux, la capacité de réponse dudit patient à un traitement par inhibiteur de point de contrôle immunitaire étant inversement proportionnelle au taux de CAF DPP4⁺ dans l'échantillon cancéreux ;
(c) la comparaison du taux de CAF DPP4⁺ dans ledit échantillon cancéreux à un taux de référence, un taux inférieur à leur taux de référence étant prédictif de la capacité de réponse dudit patient à un traitement par inhibiteur de point de contrôle immunitaire, et/ou un taux supérieur à leur taux de référence étant prédictif de l'inefficacité ou d'une efficacité moindre d'un traitement par inhibiteur de point de contrôle immunitaire sur le cancer dudit patient.

3. Méthode selon la revendication 1, ladite méthode comprenant en outre :
(a) la détection de CAF CD73⁺, et/ou CAF B7H3⁺, et/ou CAF OX40L⁺, et/ou CAF JAM2⁺ dans l'échantillon cancéreux provenant dudit patient, et
(b) la détermination du taux de CAF CD73⁺, et/ou CAF B7H3⁺, et/ou CAF OX40L⁺, et/ou CAF JAM2⁺ dans ledit échantillon cancéreux, la capacité de réponse dudit patient à un traitement par inhibiteur de point de contrôle immunitaire étant inversement proportionnelle aux taux de CAF DPP4⁺, et : CAF CD73⁺, et/ou CAF B7H3⁺, et/ou CAF OX40L⁺, et/ou CAF JAM2⁺ dans l'échantillon cancéreux ;
(c) la sélection de patients ayant un taux de CAF DPP4⁺ et : CAF CD73⁺, et/ou CAF B7H3⁺, et/ou CAF OX40L⁺, et/ou CAF JAM2⁺ inférieur à leur taux de référence comme approprié pour un traitement par inhibiteur de point de contrôle immunitaire, et/ou la sélection de patients ayant un taux de CAF DPP4⁺ et : CAF CD73⁺, et/ou CAF B7H3⁺, et/ou CAF OX40L⁺, et/ou CAF JAM2⁺ supérieur à leur taux de référence pour un traitement du cancer à l'exclusion de l'inhibiteur de point de contrôle immunitaire.

4. Inhibiteur de point de contrôle immunitaire pour l'utilisation dans le traitement d'un cancer solide chez un patient, le patient ayant été sélectionné pour le traitement par une méthode selon la revendication 1 ou 3, et le patient présentant dans un échantillon cancéreux :
(a) aucun CAF DPP4⁺ ; ou
(b) un taux de CAF DPP4⁺ inférieur à leur taux de référence.

5. Inhibiteur de point de contrôle immunitaire pour l'utilisation selon la revendication 4, le patient présentant en outre dans un échantillon cancéreux :
(a) aucun CAF CD73⁺, et/ou aucun CAF B7H3⁺, et/ou aucun CAF OX40L⁺, et/ou aucun CAF JAM2⁺, ou
(b) un ou des taux de CAF CD73⁺, et/ou CAF B7H3⁺, et/ou CAF OX40L⁺, et/ou CAF JAM2⁺ inférieur(s) à leur taux de référence.

6. Méthode selon l'une quelconque des revendications 1 à 3 ou inhibiteur de point de contrôle immunitaire pour l'utilisation selon la revendication 4 ou 5, où l'inhibiteur de point de contrôle immunitaire est choisi dans le groupe consistant en des thérapies anti-CTLA-4 (lymphocyte cytotoxique T associé à la protéine 4), inhibiteurs de PD-1 (protéine 1 de la mort cellulaire programmée), inhibiteurs de PDL1 (ligand de la mort cellulaire programmée), inhibiteurs de LAG-3 (gène 3 d'activation des lymphocytes), inhibiteurs de TIM-3 (domaine-3 contenant de la mucine et de l'immunoglobuline de lymphocyte T), inhibiteurs de TIGIT (immunorécepteur de lymphocyte T avec des domaines Ig et ITIM), inhibiteurs de BLTA (atténuateur de lymphocyte B et T), inhibiteurs IDO1, ou une combinaison de ceux-ci.

7. Méthode ou inhibiteur de point de contrôle immunitaire pour l'utilisation selon la revendication 6, où l'inhibiteur de point de contrôle immunitaire est un inhibiteur de PD-1 (protéine 1 de la mort cellulaire programmée) ou un inhibiteur de PDL1 (ligand de la mort cellulaire programmée).

8. Méthode ou inhibiteur de point de contrôle immunitaire pour l'utilisation selon la revendication 6 ou 7, où la thérapie anti-CTLA-4 est l'ipilimumab, l'inhibiteur de PD-1 est le nivolumab, le pembrolizumab, ou BGB-A-317, l'inhibiteur de PDL1 est l'azétolizumab, l'avélumab, ou le durvalumab, l'inhibiteur de LAG-3 est BMS-986016, l'inhibiteur d' IDO1 est l'épacadostat.

9. Méthode de sélection d'un patient selon l'une quelconque des revendications 1 à 3 ou 6 à 8, ou inhibiteur de point de contrôle immunitaire pour l'utilisation selon l'une quelconque des revendications 4 à 8, où le taux de CAF DPP4⁺ est le pourcentage de CAF DPP4⁺ dans l'échantillon cancéreux et le taux de référence est le pourcentage de référence.

10. Méthode de sélection d'un patient selon l'une quelconque des revendications 1 à 3 ou 6 à 8, ou inhibiteur de point de contrôle immunitaire pour l'utilisation selon l'une quelconque des revendications 4 à 8, où le pourcentage de CAF DPP4⁺ dans l'échantillon cancéreux est calculé comme étant le pourcentage de cellules CAF DPP4⁺ sur le nombre total de cellules dans l'échantillon cancéreux.

11. Méthode de sélection d'un patient selon l'une quelconque des revendications 1 à 3 ou 6 à 10, ou inhibiteur de point de contrôle immunitaire pour l'utilisation selon l'une quelconque des revendications 4 à 10, où le cancer solide est choisi dans le groupe consistant en le cancer de la prostate, le cancer du poumon, le cancer du sein, le cancer de l'estomac, le cancer des reins, le cancer des ovaires, le cancer hépatocellulaire, l'ostéosarcome, le mélanome, le cancer de l'hypopharynx, le cancer de l'esophage, le cancer de l'endomètre, le cancer du col de l'utérus, le cancer du pancréas, le cancer du foie, le cancer du colon ou colorectal, les tumeurs neuroendocrines, le cancer des muscles, le cancer des surrénales, le cancer de la thyroïde, le cancer de l'utérus, le cancer de la peau, le cancer de la vessie, le cancer de la tête et du cou, le cancer pédiatrique.

12. Méthode de sélection d'un patient selon l'une quelconque des revendications 1 à 3 ou 6 à 10, ou inhibiteur de point de contrôle immunitaire pour l'utilisation selon l'une quelconque des revendications 4 à 10, où le cancer solide est choisi dans le groupe consistant en le cancer des ovaires, le cancer du sein, le cancer du poumon, le cancer colorectal, le cancer du pancréas et le cancer pédiatrique.

13. Méthode de sélection d'un patient selon l'une quelconque des revendications 1 à 3 ou 6 à 10, ou inhibiteur de point de contrôle immunitaire pour l'utilisation selon l'une quelconque des revendications 4 à 10, où le cancer est un cancer des ovaires, de préférence un cancer des ovaires mésenchymateux, en particulier un cancer des ovaires de haut grade de type séreux.

14. Méthode de sélection d'un patient selon l'une quelconque des revendications 1 à 3 ou 6 à 10, ou inhibiteur de point de contrôle immunitaire pour l'utilisation selon l'une quelconque des revendications 4 à 10, où le cancer est un cancer du sein, de préférence un cancer du sein invasif et/ou ses métastases, en particulier ses métastases axillaires.
